# EUROPEAN PATENT APPLICATION

(11) **EP 4 331 559 A1**
(43) Date of publication of application: **06.03.2024**
(21) Application number: 22795822.0
(22) Date of filing: 26.04.2022
(51) Int. Cl.: A61K 6/887, A61C 5/00, A61C 13/00, A61K 6/62, A61K 6/76

(54) **DENTAL FILLING KIT**

(30) Priority: 26.04.2021 JP 2021074299
(71) Applicant: Kuraray Noritake Dental Inc., Kurashiki-shi, Okayama 710-0801 (JP)
(72) Inventor: MATSUURA, Ryo, Tainai-shi, Niigata 959-2653 (JP); TAKEI, Mitsuru, Tainai-shi, Niigata 959-2653 (JP)
(74) Representative: D Young & Co LLP
(86) International application number: PCT/JP2022/018984
(87) International publication number: WO 2022/230909

(57) **Abstract**

The present invention provides a dental filling kit that exhibits good cavity sealing properties even when the cured product is subjected to repeated loads after cavities deeper than 2 mm, which normally require layered filling, are filled in bulk. The present invention relates to a dental filling kit comprising: a self-adhesive dental composite resin (X) that comprises a monomer (a) having an acidic group, a monomer (b) having no acidic group, a polymerization initiator (c), and a filler (d); and a dental composite resin (Y) that comprises a monomer (b) having no acidic group, a polymerization initiator (c), and a filler (d), but does not comprise a monomer (a) having an acidic group, wherein the polymerization initiator (c) contained in the dental composite resin (Y) comprises a photopolymerization initiator (c-1), and the dental composite resin (Y) is a bulk-fill composite resin, and has depth of cure of 4 mm or more.

## Description

### TECHNICAL FIELD

The present invention relates to a dental filling kit capable of reducing the occurrence of marginal leakage even when the cured product of a bulk-fill composite resin is subjected to repeated loads in bulk-fill restorations conducted with a bulk-fill composite resin to restore cavities deeper than 2 mm, which normally require layered filling. More specifically, the present invention relates to a dental filling kit that comprises a self-adhesive dental composite resin and a bulk-fill composite resin, and that exhibits good cavity sealing properties after a cavity is filled with the bulk-fill composite resin in bulk following sealing of the cavity with the self-adhesive dental composite resin.

### BACKGROUND ART

Restorative filling materials such as filling composite resins and filling compomers, and crown restoration materials such as metal alloys, porcelain, and resin materials are typically used for the restoration of damaged tooth structures (enamel, dentin, and cementum) caused by caries and other dental defects. However, restorative filling materials and crown restoration materials (in this specification, these are also referred to collectively as "dental restoration materials") themselves do not generally possess adhesive properties to the tooth structure. Consequently, various adhesive systems with bonding agents have been used for the bonding of the tooth structure and dental restoration materials. An example of adhesive systems that are in common use is the acid etching (total etching) adhesive system, whereby the surfaces of tooth structure are etched with an acid etchant such as a phosphoric acid aqueous solution, and a bonding material, or an adhesive, is applied to bond a dental restoration material to the tooth structure.

The self-etching adhesive system, on the other hand, is an example of adhesive systems that do not use acid etchants. In the past, the mainstream in such adhesive systems has been a two-step process in which a self-etching primer containing an acidic monomer, a hydrophilic monomer, and water is first applied to surfaces of tooth structure, and a bonding material containing a crosslinkable monomer and a polymerization initiator is applied without rinsing the surfaces with water. Another type of self-etching adhesive system that has come to be commonly used in recent years is the single-step adhesive system, which uses a one-part dental adhesive (one-part bonding material) that serves as both a self-etching primer and a bonding material. The typical monomer components of the one-part bonding material are acidic monomers, hydrophilic monomers, and crosslinkable monomers, and the one-part bonding material typically uses water and hydrophilic volatile organic solvents.

Recent years have seen the development of self-adhesive dental composite resins possessing adhesive properties in the dental composite resins themselves, and some of the compositions that have already been put into practical use eliminate the use of bonding materials to reduce the number of steps in restorative treatment procedures (Patent Literatures 1 to 3). Generally, the bonding material differs from dental composite resins (such as self-adhesive dental composite resins) in that the bonding material contains a solvent (such as water or an organic solvent), and exhibits differences in filler content from dental composite resins.

Another approach that has been put into practice to reduce the number of procedural steps is what is known as bulk-fill composite resin, which has depth of cure of 4 mm or more to enable filling by a single light exposure even in deep cavities of a corresponding depth to the depth of cure.

### CITATION LIST

### Patent Literature

Patent Literature 1: JP 2004-529946 T
Patent Literature 2: JP 2017-105716 A
Patent Literature 3: JP 2018-065831 A

### SUMMARY OF INVENTION

### Technical Problem

However, the present inventors have identified, for the first time, that dental filling treatments using bonding materials and bulk-fill composite resins of related art tend to cause detachment at the bonding interface between the bonding material and the bulk-fill composite resin because of the large volume shrinkage of the bulk-fill composite resin, resulting in poor sealing of cavities (hereinafter, the properties to seal cavities will also be referred to simply as "cavity sealing properties"), especially when the cured product of the composite resin is subjected to repeated loads, though such filling treatments offer the advantage of enabling bulk filling of cavities deeper than 2 mm, which normally require layered filling. The present inventors have speculated that such low cavity sealing properties are partly due to the poor adhesive properties of the bonding material and bulk-fill composite resin because, while the bonding material is a water-containing hydrophilic composition, the bulk-fill composite resin does not usually contain water, and is hydrophobic in comparison to the bonding material.

An object of the present invention is to provide a dental filling kit that exhibits good cavity sealing properties even when the cured product is subjected to repeated loads after bulk filling of cavities deeper than 2 mm, which normally require layered filling.

### Solution to Problem

The present inventors conducted intensive studies, and found that the foregoing issues can be solved by filling a cavity with a bulk-fill composite resin in bulk after the cavity is sealed with a self-adhesive dental composite resin. This finding led to the present invention after further studies. The present invention provides a dental filling kit comprising a self-adhesive dental composite resin and a bulk-fill composite resin.

The present invention includes the following.
[1] A dental filling kit comprising:
   a self-adhesive dental composite resin (X) that comprises a monomer (a) having an acidic group, a monomer (b) having no acidic group, a polymerization initiator (c), and a filler (d); and
   a dental composite resin (Y) that comprises a monomer (b) having no acidic group, a polymerization initiator (c), and a filler (d), but does not comprise a monomer (a) having an acidic group,
   wherein the polymerization initiator (c) contained in the dental composite resin (Y) comprises a photopolymerization initiator (c-1), and
   the dental composite resin (Y) is a bulk-fill composite resin, and has depth of cure of 4 mm or more.
[2] The dental filling kit according to [1], wherein the content of the filler (d) is 50 to 90 parts by mass in total 100 parts by mass of the self-adhesive dental composite resin (X).
[3] The dental filling kit according to [1] or [2], wherein a cured product of the self-adhesive dental composite resin (X) has a flexural modulus of 6.5 GPa or less.
[4] The dental filling kit according to [3], wherein the self-adhesive dental composite resin (X) and the dental composite resin (Y) have a flexural modulus ratio ((Y)/(X)) of 0.9 to 5.0 as a ratio of a flexural modulus of a cured product of the self-adhesive dental composite resin (X) and a flexural modulus of a cured product of the dental composite resin (Y).
[5] The dental filling kit according to any one of [1] to [4], wherein the monomer (a) having an acidic group contained in the self-adhesive dental composite resin (X) comprises a monomer having a phosphoric acid group.
[6] The dental filling kit according to any one of [1] to [5], wherein the polymerization initiator (c) contained in the self-adhesive dental composite resin (X) comprises a photopolymerization initiator (c-1).
[7] The dental filling kit according to [6], wherein the photopolymerization initiator (c-1) comprises a water-soluble photopolymerization initiator (c-1a) having a solubility in 25°C water of 10 g/L or more.
[8] The dental filling kit according to any one of [1] to [7], wherein the monomer (b) having no acidic group contained in the dental composite resin (Y) comprises a (meth)acrylic acid ester (b-4) having an aromatic ring but having no hydroxyl group, and/or a (meth)acrylic acid ester (b-5) having an aromatic ring and a hydroxyl group.

### Advantageous Effects of Invention

According to the present invention, a dental filling kit can be provided that exhibits good cavity sealing properties even when the cured product is subjected to repeated loads after bulk filling of cavities deeper than 2 mm, which normally require layered filling.

### DESCRIPTION OF EMBODIMENTS

A dental filling kit of the present invention comprises a self-adhesive dental composite resin (X) and a dental composite resin (Y).

The self-adhesive dental composite resin (X) comprises a monomer (a) having an acidic group, a monomer (b) having no acidic group, a polymerization initiator (c), and a filler (d).

The dental composite resin (Y) comprises a monomer (b) having no acidic group, a polymerization initiator (c), and a filler (d), but does not comprise a monomer (a) having an acidic group.

The polymerization initiator (c) contained in the dental composite resin (Y) comprises a photopolymerization initiator (c-1). The dental composite resin (Y) is a bulk-fill composite resin. The term bulk-fill composite resin means a composite resin having depth of cure of 4 mm or more to enable bulk filling by a single light exposure in cavities of a corresponding depth to the depth of cure.

The following describes the components used in a self-adhesive dental composite resin (X) of the present invention.

### <Monomer (a) Having Acidic Group>

In view of adhesive properties to tooth structure, a monomer (a) having an acidic group is essential in a self-adhesive dental composite resin (X) of the present invention. Preferred for use in the self-adhesive dental composite resin (X) are radical monomers. Specific examples of radical monomers in monomer (a) having an acidic group include (meth)acrylate monomers, (meth)acrylamide monomers, esters, vinyl esters, and vinyl ethers of acids such as α-cyanoacrylic acid, (meth)acrylic acid, α-halogenated acrylic acid, crotonic acid, cinnamic acid, sorbic acid, maleic acid, and itaconic acid, and mono-N-vinyl derivatives and styrene derivatives. In view of curability, preferred are (meth)acrylate monomers and (meth)acrylamide monomers.

Examples of the monomer (a) having an acidic group used in the present invention include monomers having at least one acidic group such as a phosphoric acid group, a pyrophosphoric acid group, a thiophosphoric acid group, a phosphonic acid group, a carboxylic acid group, and a sulfonic acid group. The monomer (a) having an acidic group may be used alone, or two or more thereof may be used in appropriate combinations. Specific examples of the monomer (a) having an acidic group are as follows.

Examples of monomers having a phosphoric acid group include monomers having a monofunctional phosphoric acid group, such as 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 2-(meth)acryloyloxyethylphenyl hydrogen phosphate, 2-(meth)acryloyloxyethyl-(2-bromoethyl)hydrogen phosphate, 2-methacryloyloxyethyl-(4-methoxyphenyl)hydrogen phosphate, and 2-methacryloyloxypropyl-(4-methoxyphenyl)hydrogen phosphate; monomers having a bifunctional phosphoric acid group, such as glycerol phosphate di(meth)acrylate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, and 1,3-di(meth)acryloyloxypropyl dihydrogen phosphate; and acid chlorides, alkali metal salts, and amine salts of these.

Examples of monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and acid chlorides, alkali metal salts, and ammonium salts of these.

Examples of monomers having a carboxylic acid group include monofunctional (meth)acrylic acid esters having one carboxyl group or acid anhydride group thereof within the molecule, and monofunctional (meth)acrylic acid esters having a plurality of carboxyl groups or acid anhydride groups thereof within the molecule.

Examples of monofunctional monomers having one carboxyl group or acid anhydride group thereof within the molecule include (meth)acrylic acid, N-(meth)acryloylglycine, N-(meth)acryloylaspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, and N-(meth)acryloyl-4-aminosalicylic acid, and compounds derived by converting the carboxyl group of the aforementioned compounds into an acid anhydride group.

Examples of monofunctional monomers having a plurality of carboxyl groups or acid anhydride groups thereof within the molecule include 6-(meth)acryloyloxyhexane-1,1 -dicarboxylic acid, 9-(meth)acryloyloxynonane-1,1 -dicarboxylic acid, 10-(meth)acryloyloxydecane-1, 1 -dicarboxylic acid, 11-(meth)acryloyloxyundecane-1 , 1 - dicarboxylic acid, 12-(meth)acryloyloxydodecane-1, 1 -dicarboxylic acid, 13-(meth)acryloyloxytridecane-1,1-dicarboxylic acid, 4-(meth)acryloyloxyethyltrimellitate, 4-(meth)acryloyloxyethyltrimellitate anhydride, 4-(meth)acryloyloxybutyltrimellitate, 4-(meth)acryloyloxyhexyltrimellitate, 4-(meth)acryloyloxydecyltrimellitate, 2-(meth)acryloyloxyethyl-3'-(meth)acryloyloxy-2'-(3,4-dicarboxybenzoyloxy)propylsuccinate, 6-(meth)acryloyloxyethylnaphthalene-1,2,6-tricarboxylic acid anhydride, 6-(meth)acryloyloxyethylnaphthalene-2,3,6-tricarboxylic acid anhydride, 4-(meth)acryloyloxyethylcarbonylpropionoyl-1,8-naphthalic acid anhydride, and 4-(meth)acryloyloxyethylnaphthalene-1 ,8-tricarboxylic acid anhydride.

Examples of monomers having a sulfonic acid group include 2-sulfoethyl (meth)acrylate.

In view of providing good bond strength when the monomer (a) having an acidic group is used as a component of the self-adhesive dental composite resin (X), preferred among the foregoing monomers (a) having an acidic group are monomers having a phosphoric acid group, or monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyltrimellitate anhydride, 4-(meth)acryloyloxyethyltrimellitate, 11-(meth)acryloyloxyundecane-1,1-dicarboxylic acid, or a mixture of 2-methacryloyloxyethyl dihydrogen phosphate and bis(2-methacryloyloxyethyl)hydrogen phosphate, even more preferably 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, or 20-(meth)acryloyloxyicosyl dihydrogen phosphate. In view of a balance between adhesive properties and curability, 10-(meth)acryloyloxydecyl dihydrogen phosphate is particularly preferred.

The content of the monomer (a) having an acidic group in a self-adhesive dental composite resin (X) of the present invention is not particularly limited. However, in view of adhesive properties to tooth structure, the content of the monomer (a) having an acidic group in a self-adhesive dental composite resin (X) of the present invention is preferably 1 to 40 parts by mass, more preferably 2.5 to 35 parts by mass, even more preferably 5 to 30 parts by mass in total 100 parts by mass of monomers in a self-adhesive dental composite resin (X) of the present invention.

### <Monomer (b) Having no Acidic Group>

Examples of the monomer (b) having no acidic group in the present invention include asymmetrical acrylamide methacrylic acid ester compounds (b-1); hydrophobic monomers (b-2) having no acidic group and having a solubility of less than 10 g/L in 25°C water (hereinafter, also referred to simply as "hydrophobic monomers (b-2)"); and hydrophilic monomers (b-3) having no acidic group and having a solubility of 10 g/L or more in 25°C water (hereinafter, also referred to simply as "hydrophilic monomers (b-3)"). The monomer (b) having no acidic group may be used alone, or two or more thereof may be used in combination. In the present invention, compounds having no acidic group and containing an acrylamide group and a methacryloyloxy group are classified as asymmetrical acrylamide-methacrylic acid ester compounds (b-1), whereas compounds having no acidic group and excluded from asymmetrical acrylamide-methacrylic acid ester compounds (b-1) are classified as hydrophobic monomers (b-2) or hydrophilic monomers (b-3), depending on the degree of hydrophilicity.

### · Asymmetrical Acrylamide·Methacrylic Acid Ester Compound (b-1)

A certain preferred embodiment is, for example, a dental filling kit that comprises a self-adhesive dental composite resin (X) comprising an asymmetrical acrylamide methacrylic acid ester compound (b-1). In view of improvement of properties such as adhesive properties to tooth structure, and ease of adjusting the mechanical strength of the cured product within the desired range, the asymmetrical acrylamide methacrylic acid ester compound (b-1) is preferably a compound represented by the following general formula (1).

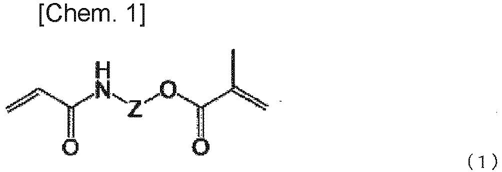

In the formula, Z is an optionally substituted linear, branched, or cyclic aliphatic group or an optionally substituted aromatic group, and the aliphatic group may be interrupted with at least one binding group selected from the group consisting of -O-, -S-, - CO-, -CO-O-, -O-CO-, -NR¹-, -CO-NR¹-, -NR¹-CO-, -CO-O-NR¹-, -O-CO-NR¹-, and -NR¹-CO-NR¹-. R¹ represents a hydrogen atom, or an optionally substituted linear or branched aliphatic group.

Z is a moiety that adjusts the hydrophilicity of the asymmetrical acrylamide methacrylic acid ester compound (b-1). The optionally substituted aliphatic group represented by Z may be either a saturated aliphatic group (an alkylene group, a cycloalkylene group (for example, such as a 1,4-cyclohexylene group)), or an unsaturated aliphatic group (an alkenylene group, an alkynylene group). In view of availability or ease of production, and chemical stability, preferred are saturated aliphatic groups (alkylene groups). In view of adhesive properties to tooth structure and polymerization curability, Z is preferably a C₁ to C₈ aliphatic group, more preferably a C₁ to C₄ aliphatic group, even more preferably a C₂ to C₄ aliphatic group. Examples of the C₁ to C₈ alkylene group include a methylene group, an ethylene group, an n-propylene group, an isopropylene group, and an n-butylene group.

Examples of the optionally substituted aromatic group represented by Z include an arylene group, and an aromatic heterocyclic group. The aromatic group is preferably an arylene group rather than an aromatic heterocyclic group. The hetero ring in the aromatic heterocyclic group is normally unsaturated. The aromatic hetero ring is preferably a five-membered ring or a six-membered ring. Preferably, the arylene group is, for example, a phenylene group. Examples of the hetero ring in the aromatic heterocyclic group include a furan ring, a thiophene ring, a pyrrole ring, an oxazole ring, an isoxazole ring, a thiazole ring, an isothiazole ring, an imidazole ring, a pyrazole ring, a furazan ring, a triazole ring, a pyran ring, a pyridine ring, a pyridazine ring, a pyrimidine ring, a pyrazine ring, and a 1,3,5-triazine ring. The aromatic group is particularly preferably a phenylene group.

The aliphatic group represented by R¹ may be either a saturated aliphatic group (an alkyl group) or an unsaturated aliphatic group (an alkenyl group, an alkynyl group). In view of availability or ease of production, and chemical stability, preferred are saturated aliphatic groups (alkyl groups). Examples of the linear or branched alkyl group represented by R¹ include methyl, ethyl, n-propyl, isopropyl, n-butyl, isobutyl, sec-butyl, tert-butyl, n-pentyl, isopentyl, sec-pentyl, neopentyl, tert-pentyl, 1-ethylpropyl, hexyl, isohexyl, 1,1-dimethylbutyl, 2,2-dimethylbutyl, 3,3-dimethylbutyl, and 2-ethylbutyl. Preferred examples include methyl, ethyl, n-propyl, isopropyl, n-butyl, isobutyl, sec-butyl, and tert-butyl.

R¹ is preferably a hydrogen atom, or a C₁ to C₈ aliphatic group, more preferably a hydrogen atom, or an optionally substituted C₁ to C₄ alkyl group, even more preferably a hydrogen atom, or an optionally substituted C₁ to C₃ alkyl group.

When the aliphatic group represented by Z is interrupted by the above binding group, the number of binding groups is not particularly limited, and may be about 1 to 10, preferably 1, 2, or 3, more preferably 1 or 2. In the formula (1), the aliphatic group represented by Z is preferably one that is not contiguously interrupted by the binding group. That is, the aliphatic group represented by Z is preferably one in which the binding groups are not adjacent to each other. The binding group is more preferably at least one selected from the group consisting of -O-, -S-, -CO-, -CO-O-, -O-CO-, -NH-, - CO-NH-, -NH-CO-, -CO-O-NH-, -O-CO-NH-, and -NH-CO-NH-, particularly preferably at least one selected from the group consisting of -O-, -S-, -CO-, -NH-, -CO-NH-, and -NH-CO-.

Examples of the substituents in Z include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group.

Specific examples of the asymmetric acrylamide methacrylic acid ester compound (b-1) include, but are not particularly limited to, the following.

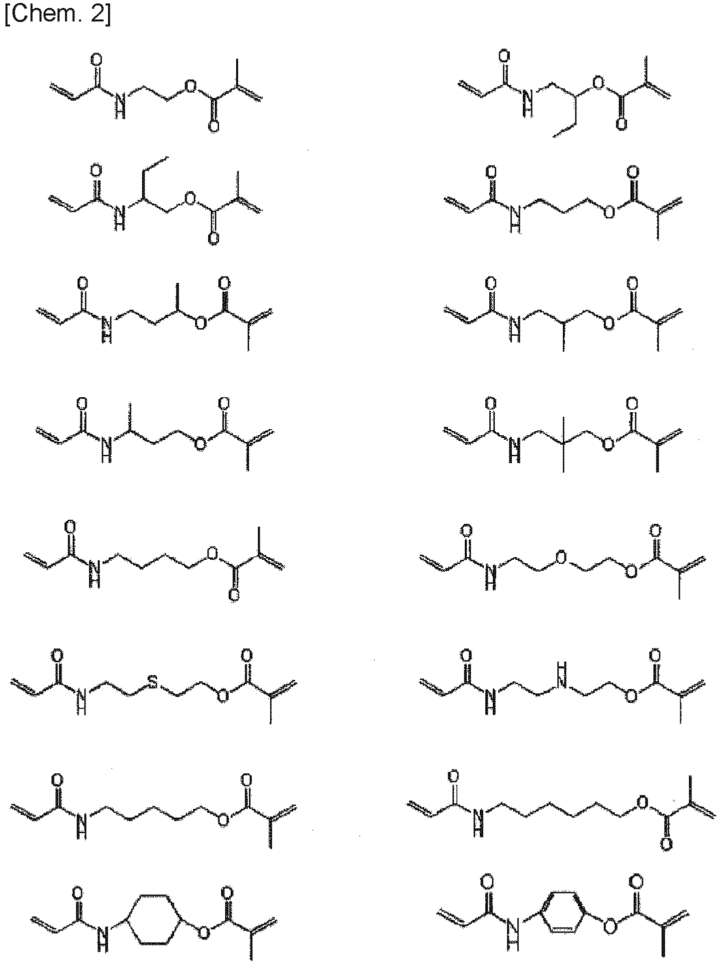

In view of adhesive properties to tooth structure and polymerization curability, preferred are asymmetric acrylamide-methacrylic acid ester compounds in which Z is an optionally substituted C₂ to C₄ linear or branched aliphatic group, more preferably N-methacryloyloxyethylacrylamide (commonly known as MAEA), N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide. In view of the high hydrophilicity concerning penetration into the collagen layer of dentin, even more preferred are MAEA, and N-methacryloyloxypropylacrylamide.

The asymmetric acrylamide-methacrylic acid ester compound (b-1) may be contained alone, or two or more thereof may be contained in combination. The content of asymmetric acrylamide-methacrylic acid ester compound (b-1) is not particularly limited, as long as the present invention can exhibit its effects. However, the content of asymmetric acrylamide-methacrylic acid ester compound (b-1) is preferably 1 to 60 parts by mass, more preferably 2 to 45 parts by mass, even more preferably 3 to 30 parts by mass, particularly preferably 5 to 25 parts by mass in total 100 parts by mass of monomers in a self-adhesive dental composite resin (X) of the present invention.

### · Hydrophobic Monomer (b-2) Having no Acidic Group

A certain preferred embodiment is, for example, a dental filling kit that comprises a self-adhesive dental composite resin (X) comprising a hydrophobic monomer (b-2) having no acidic group. The hydrophobic monomer (b-2) having no acidic group improves properties such as ease of handling of the self-adhesive dental composite resin (X), and the mechanical strength of the cured product. The hydrophobic monomer (b-2) is preferably a radical monomer having no acidic group but having a polymerizable group. In view of ease of radical polymerization, preferred as the polymerizable group are (meth)acryloyloxy groups and/or (meth)acrylamide groups. The hydrophobic monomer (b-2) means a monomer that has no acidic group, and does not correspond to the asymmetric acrylamide-methacrylic acid ester compound (b-1), and that has a solubility of less than 10 g/L in 25°C water. Examples of the hydrophobic monomer (b-2) include crosslinkable monomers such as aromatic bifunctional monomers, aliphatic bifunctional monomers, and tri- and higher-functional monomers.

Examples of the aromatic bifunctional monomers include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred are 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-methacryloyloxypolyethoxyphenyl)propane (with an average of 2.6 moles of ethoxy group added, commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic bifunctional monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, and 2,2,4-trimethylhexamethylenebis(2-carbamoyloxyethyl)di(meth)acrylate. Preferred are triethylene glycol diacrylate, triethylene glycol dimethacrylate (commonly known as 3G), neopentyl glycol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as UDMA), and 1,10-decanediol dimethacrylate (commonly known as DD).

Examples of the tri- and higher-functional monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. Preferred is N,N-(2,2,4-trimethylhexamethylene)bis[2-(aminocarboxy)propane-1,3-diol]tetramethacrylate.

In view of the mechanical strength of the cured product and ease of handling, preferred among the foregoing hydrophobic monomers (b-2) are aromatic bifunctional monomers, and aliphatic bifunctional monomers. Preferred as the aromatic bifunctional monomers are Bis-GMA and D-2.6E. Preferred as the aliphatic bifunctional monomers are glycerol di(meth)acrylate, 3G, neopentyl glycol di(meth)acrylate, 1,6-hexanediol di(meth)acrylate, DD, 1,2-bis(3-methacryloyloxy-2-hydroxypropoxy)ethane, and UDMA.

In view of providing good adhesive properties to tooth structure when the hydrophobic monomer (b-2) is used as a component of the self-adhesive dental composite resin (X) (composition), the hydrophobic monomer (b-2) is more preferably Bis-GMA, D-2.6E, 3G, UDMA, or DD, even more preferably D-2.6E, 3G, or Bis-GMA.

The hydrophobic monomer (b-2) may be contained alone, or two or more thereof may be used in combination. The content of the hydrophobic monomer (b-2) in a self-adhesive dental composite resin (X) of the present invention is preferably 20 to 98 parts by mass, more preferably 40 to 95 parts by mass, even more preferably 60 to 92 parts by mass in total 100 parts by mass of monomers in a self-adhesive dental composite resin (X) of the present invention. When the content of hydrophobic monomer (b-2) is at or below the foregoing upper limits, a decrease of adhesion due to reduced wettability of the self-adhesive dental composite resin (X) to tooth structure can more easily be reduced, whereas the cured product can more easily exhibit the desired mechanical strength when the content of hydrophobic monomer (b-2) is at or above the foregoing lower limits.

### · Hydrophilic Monomer (b-3) Having no Acidic Group

A certain preferred embodiment is, for example, a dental filling kit that comprises a self-adhesive dental composite resin (X) comprising a hydrophilic monomer (b-3) having no acidic group. The hydrophilic monomer (b-3) improves the wettability of self-adhesive dental composite resin (X) to tooth structure. The hydrophilic monomer (b-3) is preferably a radical monomer having no acidic group but having a polymerizable group. In view of ease of radical polymerization, preferred as the polymerizable group are (meth)acryloyloxy groups and/or (meth)acrylamide groups. The hydrophilic monomer (b-3) means a monomer that has no acidic group, and does not correspond to the asymmetric acrylamide-methacrylic acid ester compound (b-1), and that has a solubility of 10 g/L or more in 25°C water. The hydrophilic monomer (b-3) is preferably one having a solubility of 30 g/L or more in 25°C water, more preferably one that can dissolve in water in any proportions at 25°C. The hydrophilic monomer is preferably one having a hydrophilic group such as a hydroxyl group, an oxymethylene group, an oxyethylene group, an oxypropylene group, or an amide group. Examples of the hydrophilic monomer (b-3) include hydrophilic monofunctional (meth)acrylate monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and polyethylene glycol di(meth)acrylate (with nine or more oxyethylene groups); and hydrophilic monofunctional (meth)acrylamide monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, and N,N-diethylacrylamide.

In view of adhesive properties to tooth structure, preferred as hydrophilic monomer (b-3) are 2-hydroxyethyl (meth)acrylate, 2,3-dihydroxypropyl (meth)acrylate, and hydrophilic monofunctional (meth)acrylamide monomers, more preferably 2-hydroxyethyl (meth)acrylate, N,N-dimethylacrylamide, and N,N-diethylacrylamide. The hydrophilic monomer (b-3) may be contained alone, or two or more thereof may be used in combination.

The content of the hydrophilic monomer (b-3) in a self-adhesive dental composite resin (X) of the present invention ranges preferably from 0 to 50 parts by mass, more preferably from 0 to 40 parts by mass, even more preferably from 0 to 30 parts by mass in total 100 parts by mass of monomers in a self-adhesive dental composite resin (X) of the present invention. The content of hydrophilic monomer (b-3) may be 0 part by mass in total 100 parts by mass of monomers in a self-adhesive dental composite resin (X) of the present invention. When the content of the hydrophilic monomer (b-3) in a self-adhesive dental composite resin (X) of the present invention is at or above these lower limits, it is easier to produce a sufficient adhesion improving effect, whereas the cured product can more easily exhibit the desired mechanical strength when the content of hydrophilic monomer (b-3) is at or below the foregoing upper limits.

The content of the monomer (b) having no acidic group in the self-adhesive dental composite resin (X) is preferably 60 to 99 parts by mass, more preferably 65 to 97.5 parts by mass, even more preferably 70 to 95 parts by mass in total 100 parts by mass of monomers in a self-adhesive dental composite resin (X) of the present invention. In view of adhesive properties to tooth structure, the hydrophobic monomer (b-2) and hydrophilic monomer (b-3) have a mass ratio ((b-2):(b-3)) of preferably 10:0 to 1:2, more preferably 10:0 to 1:1, even more preferably 10:0 to 2:1.

A certain preferred embodiment is, for example, a self-adhesive dental composite resin (X) that is essentially free of a bi- or higher functional (meth)acrylamide monomer. Another certain preferred embodiment is, for example, a self-adhesive dental composite resin (X) that is essentially free of a phosphoric acid hydrogen diester group-containing monomer. The phosphoric acid hydrogen diester group-containing monomer has a (meth)acryloyloxy group and/or a (meth)acrylamide group. In the present invention, "being essentially free of a polymerizable compound" means that the content of the polymerizable compound is less than 0.5 parts by mass, preferably less than 0.1 parts by mass, more preferably less than 0.01 parts by mass in total 100 parts by mass of monomers contained in a composition of the self-adhesive dental composite resin (X). The polymerizable compound content may be 0 part by mass. The content of the polymerizable compound that is essentially absent may be less than 0.5 mass%, or less than 0.1 mass% in the whole composition of the self-adhesive dental composite resin (X).

### <Polymerization Initiator (c)>

The polymerization initiator (c) comprises a photopolymerization initiator (c-1) or a chemical polymerization initiator (c-2). These may be contained alone, or two or more thereof may be used in combination. In view of providing the desired mechanical strength for the cured product with greater ease, a certain preferred embodiment is, for example, a dental filling kit in which the polymerization initiator (c) contained in the self-adhesive dental composite resin (X) comprises a photopolymerization initiator (c-1).

The photopolymerization initiator (c-1) can be classified into a water-soluble photopolymerization initiator (c-1a) and a water-insoluble photopolymerization initiator (c-1b). The photopolymerization initiator (c-1) may be solely a water-soluble photopolymerization initiator (c-1a) or a water-insoluble photopolymerization initiator (c-1 b), or may be a combination of a water-soluble photopolymerization initiator (c-1a) and a water-insoluble photopolymerization initiator (c-1b). Preferably, a water-soluble photopolymerization initiator (c-1a) and a water-insoluble photopolymerization initiator (c-1b) are used in combination.

### · Water-Soluble Photopolymerization Initiator (c-1a)

With a water-soluble photopolymerization initiator (c-1a), a high bond strength can be achieved by improving polymerization curability at the interface with hydrophilic tooth surfaces, providing good cavity sealing properties, and improving cavity sealing properties even under repeated loading. The water-soluble photopolymerization initiator (c-1a) has a solubility in 25°C water of 10 g/L or more, preferably 15 g/L or more, more preferably 20 g/L or more, even more preferably 25 g/L or more. By having a solubility of 10 g/L or more in 25°C water, the water-soluble photopolymerization initiator (c-1a) can sufficiently dissolve in water within the tooth structure at the bonding interface, and more easily exhibits the polymerization promoting effect. A certain preferred embodiment is, for example, a dental filling kit in which the polymerization initiator (c) contained in the self-adhesive dental composite resin (X) comprises a water-soluble photopolymerization initiator (c-1a) having a solubility in 25°C water of 10 g/L or more.

Examples of the water-soluble photopolymerization initiator (c-1a) include water-soluble thioxanthones; water-soluble acylphosphine oxides; and α-hydroxyalkylacetophenones, for example, such as compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having -OCH₂COO⁻Na⁺ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having -OCH₂COO⁻Na⁺ introduced into the phenyl group of 2-hydroxy-2-methyl-1-phenylpropan-1-one. Other examples of the water-soluble photopolymerization initiator (c-1a) include quaternary ammonium compounds prepared by quaternization of the amino group of α-aminoalkylphenones, such as 2-methyl-1-[4-(methylthio)phenyl]-2-morpholinopropan-1 -one, and 2-benzyl-2-(dimethylamino)-1-[(4-morpholino)phenyl]-1-butanon.

The water-soluble thioxanthones may be any of, for example, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminium chloride, 2-hydroxy-3-(9-oxo-9H-thioxanthen-2-yloxy)-N,N,N-trimethyl-1 -propaneaminium chloride, 2-hydroxy-3-(3,4-dimethyl-9-oxo-9H-thioxanthen-2-yloxy)-N, N, N-trimethyl-1-propaneaminium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propaneaminium chloride.

Examples of the water-soluble acylphosphine oxides include acylphosphine oxides represented by the following general formula (2) or (3).

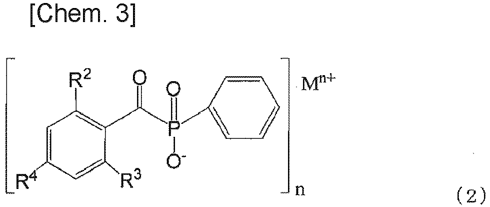

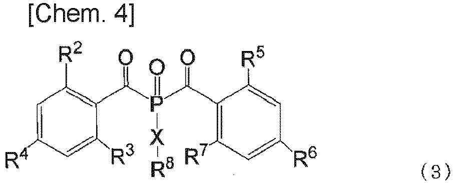

In formulae (2) and (3), R², R³, R⁴, R⁵, R⁶, and R⁷ are each independently a C₁ to C₄ linear or branched alkyl group or a halogen atom, M is a hydrogen ion, an alkali metal ion, an alkali earth metal ion, a magnesium ion, a pyridinium ion (the pyridine ring may have a substituent), or an ammonium ion represented by HN⁺R⁹R¹⁰R¹¹ (where R⁹, R¹⁰, and R¹¹ are each independently an organic group or a hydrogen atom), n is 1 or 2, X is a C₁ to C₄ linear or branched alkylene group, and R⁸ represents - CH(CH₃)COO(C₂H₄O)_{P}CH₃, where p represents an integer of 1 to 1,000.

The alkyl group represented by R², R³, R⁴, R⁵, R⁶, and R⁷ is not particularly limited, as long as it is a C₁ to C₄ linear or branched alkyl group. Examples include methyl, ethyl, n-propyl, isopropyl, n-butyl, isobutyl, sec-butyl, 2-methylpropyl, and tert-butyl. The alkyl group represented by R², R³, R⁴, R⁵, R⁶, and R⁷ is preferably a C₁ to C₃ linear alkyl group, more preferably methyl or ethyl, even more preferably methyl. Examples of X include methylene, ethylene, n-propylene, isopropylene, and n-butylene. X is preferably a C₁ to C₃ linear alkylene group, more preferably methylene or ethylene, even more preferably methylene.

Examples of the substituent of the pyridine ring when M is a pyridinium ion include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group. Preferably, M is an alkali metal ion, an alkali earth metal ion, a magnesium ion, a pyridinium ion (the pyridine ring may have a substituent), or an ammonium ion represented by HN⁺R⁹R¹⁰R¹¹ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion. The organic group represented by R⁹, R¹⁰, and R¹¹ may be the same group exemplified above for the substituent of the pyridine ring (excluding a halogen atom).

In view of storage stability and shade stability in a composition of the self-adhesive dental composite resin (X), particularly preferred are compounds in which R², R³, R⁴, R⁵, R⁶, and R⁷ are all methyl groups. Examples of ammonium ions include ammonium ions derived from various amines. Examples of the amines include ammonia, trimethylamine, diethylamine, dimethylaniline, ethylenediamine, triethanolamine, N,N-dimethylaminomethacrylate, 4-(N,N-dimethylamino)benzoic acid and alkyl esters thereof, 4-(N,N-diethylamino)benzoic acid and alkyl esters thereof, and N,N-bis(2-hydroxyethyl)-p-toluidine.

In view of adhesive properties, p in R⁸ is preferably 1 or more, more preferably 2 or more, even more preferably 3 or more, particularly preferably 4 or more, and is preferably 1,000 or less, more preferably 100 or less, even more preferably 75 or less, particularly preferably 50 or less.

Particularly preferred among these water-soluble acylphosphine oxides are lithium phenyl(2,4,6-trimethylbenzoyl)phosphinate, sodium phenyl(2,4,6-trimethylbenzoyl)phosphinate, and compounds represented by general formula (3) and in which the moiety corresponding to the group represented by R⁸ is synthesized from polyethylene glycol methyl ether methacrylate having a molecular weight of 950.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides. The water-soluble photopolymerization initiator (c-1a) may be used alone, or two or more thereof may be used in combination.

The water-soluble photopolymerization initiator (c-1a) may be dissolved in the self-adhesive dental composite resin (X), or may be dispersed in the form of a powder in a composition of the self-adhesive dental composite resin (X).

When the water-soluble photopolymerization initiator (c-1a) is dispersed in the form of a powder, the average particle diameter is preferably 500 µm or less, more preferably 100 µm or less, even more preferably 50 µm or less because the water-soluble photopolymerization initiator (c-1a) tends to settle when the average particle diameter of the powder is excessively large. The average particle diameter is preferably 0.01 µm or more because the specific surface area of the powder overly increases, and the amount of powder that can be dispersed in a composition of the self-adhesive dental composite resin (X) decreases when the average particle diameter of the powder is excessively small. Taken together, the average particle diameter of water-soluble photopolymerization initiator (c-1a) preferably ranges from 0.01 to 500 µm, more preferably 0.01 to 100 µm, even more preferably 0.01 to 50 µm.

The average particle diameter of a powder of water-soluble photopolymerization initiator (c-1a) can be determined by taking an electron micrograph of at least 100 particles, and calculating the volume average particle diameter from the captured image after an image analysis with image-analyzing particle size distribution measurement software (Mac-View, manufactured by Mountech Co., Ltd.).

The shape of the water-soluble photopolymerization initiator (c-1a) when it is dispersed in the form of a powder is not particularly limited, and may be any of various shapes, including, for example, spherical, stylus, plate-like, and crushed shapes. The water-soluble photopolymerization initiator (c-1a) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the self-adhesive dental composite resin (X) obtained, the content of water-soluble photopolymerization initiator (c-1a) is preferably 0.01 to 20 parts by mass relative to total 100 parts by mass of monomers in a self-adhesive dental composite resin (X) of the present invention. In view of adhesiveness to tooth structure, the content of water-soluble photopolymerization initiator (c-1a) is more preferably 0.05 to 10 parts by mass, even more preferably 0.1 to 5 parts by mass relative to total 100 parts by mass of monomers in a self-adhesive dental composite resin (X) of the present invention. When the content of water-soluble photopolymerization initiator (c-1a) is at or above these lower limits, polymerization can sufficiently proceed at the bonding interface, making it easier to provide a sufficient bond strength. When the content of water-soluble photopolymerization initiator (c-1a) is at or below the foregoing upper limits, it is easier to provide a sufficient bond strength.

### . Water-Insoluble Photopolymerization Initiator (c-1b)

In view of curability, a self-adhesive dental composite resin (X) of the present invention preferably comprises a water-insoluble photopolymerization initiator (c-1b) having a solubility of less than 10 g/L in 25°C water (hereinafter, also referred to as "water-insoluble photopolymerization initiator (c-1b)"), in addition to the water-soluble photopolymerization initiator (c-1a). The water-insoluble photopolymerization initiator (c-1b) used in the present invention may be a known water-insoluble photopolymerization initiator. The water-insoluble photopolymerization initiator (c-1b) may be contained alone, or two or more thereof may be contained in combination.

Examples of the water-insoluble photopolymerization initiator (c-1b) include (bis)acylphosphine oxides (other than those exemplified for the water-soluble photopolymerization initiator (c-1a)), thioxanthones, ketals, α-diketones, coumarins, anthraquinones, benzoin alkyl ether compounds, and α-aminoketone compounds.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarins include compounds mentioned in JP H09-3109 A and JP H10-245525 A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano[6,7,8-ij]quinolizin-11-one.

Particularly preferred among these coumarins are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Example of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

Among these examples, the water-insoluble photopolymerization initiator (c-1b) is preferably at least one selected from the group consisting of a (bis)acylphosphine oxide, an α-diketone, and a coumarin. In this way, a self-adhesive dental composite resin (X) can be obtained that has excellent photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (c-1b) is not particularly limited. However, in view of curability and other properties of the composition of self-adhesive dental composite resin (X) obtained, the content of water-insoluble photopolymerization initiator (c-1b) is preferably 0.01 to 10 parts by mass, more preferably 0.05 to 7 parts by mass, even more preferably 0.1 to 5 parts by mass relative to total 100 parts by mass of monomers in a self-adhesive dental composite resin (X) of the present invention. When the content of water-insoluble photopolymerization initiator (c-1b) is at or below these upper limits, it is easier to obtain a sufficient bond strength even when the water-insoluble photopolymerization initiator (c-1b) itself has low polymerization performance, in addition to reducing a possibility of precipitation of the water-insoluble photopolymerization initiator (c-1b) itself from the self-adhesive dental composite resin (X).

When the water-soluble photopolymerization initiator (c-1a) and the water-insoluble photopolymerization initiator (c-1b) are used in combination, the mass ratio (c-1 a): (c-1b) of water-soluble photopolymerization initiator (c-1a) and water-insoluble photopolymerization initiator (c-1b) in the present invention is preferably 10:1 to 1:10, more preferably 7:1 to 1:7, even more preferably 5:1 to 1:5, particularly preferably 3:1 to 1:3. When the fraction of water-soluble photopolymerization initiator (c-1a) in the mass ratio exceeds 10:1, the curability of the self-adhesive dental composite resin (X) itself decreases, and the composition may have difficulty in exhibiting high bond strength. When the fraction of water-insoluble photopolymerization initiator (c-1b) in the mass ratio exceeds 1:10, the self-adhesive dental composite resin (X) may have difficulty in exhibiting high bond strength as a result of insufficient promotion of polymerization at the bonding interface, though the curability of the composition itself can increase.

In view of considerations such as the curability of the self-adhesive dental composite resin (X) obtained, the content of photopolymerization initiator (c-1) is preferably 0.01 to 20 parts by mass relative to total 100 parts by mass of monomers in a self-adhesive dental composite resin (X) of the present invention. In view of adhesiveness to tooth structure, the content of photopolymerization initiator (c-1) is more preferably 0.05 to 10 parts by mass, even more preferably 0.1 to 5 parts by mass, particularly preferably 0.15 to 2.5 parts by mass. When the content of photopolymerization initiator (c-1) is at or above the foregoing lower limits, it is easier to provide a sufficient bond strength by allowing polymerization to sufficiently proceed at the bonding interface. When the content of photopolymerization initiator (c-1) is at or below the foregoing upper limits, it is easier to provide a sufficient bond strength.

### . Chemical Polymerization Initiator (c-2)

A self-adhesive dental composite resin (X) of the present invention may additionally comprise a chemical polymerization initiator (c-2). Preferred for use as chemical polymerization initiator (c-2) are organic peroxides. The organic peroxides used as chemical polymerization initiator (c-2) are not particularly limited, and known organic peroxides may be used. Typical examples of such organic peroxides include ketone peroxides, hydroperoxides, diacyl peroxides, dialkyl peroxides, peroxyketals, peroxyesters, and peroxydicarbonates. Specific examples of these organic peroxides include those mentioned in WO2008/087977. The chemical polymerization initiator (c-2) may be used alone, or two or more thereof may be used in combination.

### <Filler (d)>

A self-adhesive dental composite resin (X) of the present invention comprises a filler (d), in order to adjust the ease of handling, and to increase the mechanical strength of the cured product. Examples of such fillers include an inorganic filler (d-1), an organic-inorganic composite filler (d-2), and an organic filler (d-3). The filler (d) may be used alone, or two or more thereof may be used in combination.

Examples of the materials of inorganic filler (d-1) include quartz, silica, alumina, composite oxides (for example, silica-titania-barium oxide, silica-zirconia, silica-titania, silica-alumina, silica-alumina-zirconia), various types of glasses (for example, glasses containing silica as a main component, optionally with oxides of elements such as heavy metals, boron, zirconium, titanium, and aluminum; examples of such glasses include fused silica, lanthanum glass, borosilicate glass, soda glass, strontium glass, glass-ceramics, aluminosilicate glass, strontium boroaluminosilicate glass, fluoroaluminosilicate glass, calcium fluoroaluminosilicate glass, strontium fluoroaluminosilicate glass, strontium calcium fluoroaluminosilicate glass, and barium glass (e.g., barium silicate glass, barium boroaluminosilicate glass, barium fluoroaluminosilicate glass)), ytterbium oxide, and silica-coated ytterbium fluoride. These may be used alone, or two or more thereof may be used as a mixture. In view of providing superior mechanical strength and superior transparency to the self-adhesive dental composite resin (X) obtained, preferred are quartz, silica, silica-zirconia composite oxide, barium glass, ytterbium oxide, and silica-coated ytterbium fluoride, more preferably quartz, silica, silica-zirconia composite oxide, barium glass, and silica-coated ytterbium fluoride. In view of considerations such as the ease of handling and mechanical strength of the self-adhesive dental composite resin (X) obtained, the average particle diameter of the inorganic filler (d-1) is preferably 0.001 to 50 µm, more preferably 0.001 to 10 µm. In this specification, the average particle diameter of inorganic filler means the average particle diameter before surface treatment when the inorganic filler is subjected to a surface treatment, as will be described. A certain preferred embodiment is, for example, a dental filling kit in which the filler (d) contained in the self-adhesive dental composite resin (X) is an inorganic filler (d-1).

The shape of inorganic filler (d-1) is not particularly limited, and the particle diameter of filler may be appropriately selected for use. For example, the inorganic filler (d-1) may be an irregularly shaped filler or a spherical filler. In view of improving the mechanical strength of a cured product of the self-adhesive dental composite resin (X), the inorganic filler used is preferably a spherical filler. Here, the spherical filler represents particles that appear round in shape when observed in a unit field of an electron micrograph, and that have an average uniformity of 0.6 or more as calculated by dividing the diameter of a particle perpendicular to its maximum diameter by the maximum diameter. The spherical filler has an average particle diameter of preferably 0.05 to 5 µm. When the average particle diameter is at or above the foregoing lower limits, it is easier to provide a sufficient filling rate for the spherical filler in the self-adhesive dental composite resin (X), and desired mechanical strength can be obtained with greater ease. When the average particle diameter is at or below the foregoing upper limits, the spherical filler is less likely to decrease its surface area, making it easier to provide a desired mechanical strength in the cured product of self-adhesive dental composite resin (X).

In order to adjust the flowability of the self-adhesive dental composite resin (X), or to more easily produce a desired mechanical strength in the cured product, the inorganic filler (d-1) may be used after an optional surface treatment with a known surface treatment agent such as a silane coupling agent. For example, the hydroxyl groups present on the surface of inorganic filler (d-1) may be surface treated with a silane coupling agent to obtain an inorganic filler having its hydroxyl groups surface treated. Examples of the surface treatment agent include vinyltrimethoxysilane, vinyltriethoxysilane, vinyltrichlorosilane, vinyl tri(β-methoxyethoxy)silane, γ-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, and γ-aminopropyltriethoxysilane.

The surface treatment can be carried out using any known method, including, for example, a method that adds the surface treatment agent by spraying it while vigorously stirring the inorganic filler (d-1), a method that disperses or dissolves the inorganic filler (d-1) and surface treatment agent in a suitable solvent, and removes the solvent, and a method in which the alkoxy group of the surface treatment agent is transformed into a silanol group by hydrolysis with an acid catalyst in an aqueous solution, and the silanol group is allowed to adhere to the inorganic filler surface in the aqueous solution before removal of water. In all of these methods, the reaction between the surface of inorganic filler (d-1) and the surface treatment agent can proceed to completion to enable a surface treatment by applying heat in a temperature range of typically 50 to 150°C. The amount of surface treatment agent is not particularly limited. For example, the amount of surface treatment agent is preferably 0.05 to 100 parts by mass, more preferably 0.10 to 50 parts by mass relative to 100 parts by mass of inorganic filler (d-1) before surface treatment.

The organic-inorganic composite filler (d-2) used in the present invention is a filler obtained by adding a monomer to the inorganic filler (d-1), polymerizing the mixture in paste form, and pulverizing the polymerized filler. The organic-inorganic composite filler (d-2) refers to a filler comprising the inorganic filler (d-1) and a polymer of a polymerizable monomer. The organic-inorganic composite filler (d-2) may be, for example, a TMPT filler (a filler obtained by mixing trimethylolpropane methacrylate and a silica filler, and pulverizing the mixture after polymerization). The shape of the organic-inorganic composite filler (d-2) is not particularly limited, and the particle diameter of filler may be appropriately selected for use. In view of properties such as the ease of handling and mechanical strength of the composition of self-adhesive dental composite resin (X) obtained, the organic-inorganic composite filler (d-2) has an average particle diameter of preferably 0.001 to 50 µm, more preferably 0.001 to 10 µm.

Examples of the materials of the organic filler (d-3) include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of organic filler (d-3) is not particularly limited, and the particle diameter of filler may be appropriately selected for use. In view of considerations such as the ease of handling and mechanical strength of the self-adhesive dental composite resin (X) obtained, the average particle diameter of the organic filler (d-3) is preferably 0.001 to 50 µm, more preferably 0.001 to 10 µm.

In this specification, the average particle diameter of filler can be determined by a laser diffraction scattering method or by observing particles with an electron microscope. Specifically, a laser diffraction scattering method is more convenient for the measurement of particles having a particle size of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View, manufactured by Mountech Co., Ltd.). Here, the particle diameter is determined as an arithmetic mean value of the maximum and minimum lengths of particles, and the average primary particle diameter is calculated from the number of particles and the particle diameter.

In a self-adhesive dental composite resin (X) of the present invention, it is preferable to use a mixture or a combination of two or more fillers of different materials, different particle size distributions, and different forms. By combining two or more types of fillers, the fillers can interact with the monomer or with the other fillers at an increased number of points, in addition to densely packing themselves. Depending on the type of filler, it is also possible to control paste fluidity in the presence or absence of a shear force. In view of the ease of handling and paste properties of a self-adhesive dental composite resin (X) of the present invention, the filler (d) is preferably a combination (I) of a filler (d-i) having an average particle diameter of 1 nm or more and less than 0.1 µm, and a filler (d-ii) having an average particle diameter of 0.1 µm or more and 1 µm or less; a combination (II) of a filler (d-i) having an average particle diameter of 1 nm or more and less than 0.1 µm, and a filler (d-iii) having an average particle diameter of more than 1 µm and 10 µm or less; a combination (III) of a filler (d-i) having an average particle diameter of 1 nm or more and less than 0.1 µm, a filler (d-ii) having an average particle diameter of 0.1 µm or more and 1 µm or less, and a filler (d-iii) having an average particle diameter of more than 1 µm and 10 µm or less; or a combination (IV) of fillers (d-ii) having an average particle diameter of 0.1 µm or more and 1 µm or less. In view of paste properties, cavity sealing properties, and ease of providing a desirable mechanical strength in the cured product, the combinations (I), (II), and (III) are more preferred, and the combinations (I) and (II) are even more preferred. The combination (IV) of fillers (d-ii) having an average particle diameter of 0.1 µm or more and 1 µm or less means an embodiment that comprises two types of fillers (d-ii) of different average particle diameters between 0.1 µm and 1 µm. The fillers (d) of different particle diameters may include other types of fillers, provided that the fillers (d) have the foregoing combinations. Unintended inclusion of non-filler particles as impurities is acceptable to such an extent that it does not hinder the effectiveness of the present invention.

The content of filler (d) is not particularly limited. In view of the mechanical strength of the cured product and adhesive properties to tooth structure, the content of filler (d) is preferably 50 to 90 parts by mass, more preferably 55 to 85 parts by mass, even more preferably 60 to 80 parts by mass in total 100 parts by mass of self-adhesive dental composite resin (X). The content of filler (d) relative to total 100 parts by mass of monomers may fall in the ranges represented by the preferred fractions of components presented below.

In certain embodiments, the content of filler (d-i) having an average particle diameter of 1 nm or more and less than 0.1 µm is preferably 0.001 to 10 parts by mass, more preferably 0.05 to 8 parts by mass, even more preferably 0.01 to 5 parts by mass in total 100 parts by mass of the self-adhesive dental composite resin (X).

The form of a self-adhesive dental composite resin (X) of the present invention is not particularly limited, and may be, for example, a two-pack type (two-paste type). However, in view of workability, a self-adhesive dental composite resin (X) of the present invention is preferably of a one-pack type (one-paste type) with fully pre-mixed components.

A method of production of self-adhesive dental composite resin (X) is not particularly limited, and a self-adhesive dental composite resin (X) of the present invention can be produced with ease using a method known to a person skilled in the art (for example, by mixing the necessary components).

### <Polymerization Accelerator (e)>

A self-adhesive dental composite resin (X) of the present invention may use a polymerization accelerator (e) with the polymerization initiator (c). Examples of the polymerization accelerator (e) that can be used in the present invention include amines, sulfinic acid and salts thereof, borate compounds, barbituric acid compounds, triazine compounds, copper compounds, tin compounds, vanadium compounds, halogen compounds, aldehydes, thiol compounds, sulfites, bisulfites, and thiourea compounds.

The amines used as polymerization accelerator (e) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the self-adhesive dental composite resin (X), preferred for use are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart excellent curability to the self-adhesive dental composite resin (X), it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of sulfinic acid and salts thereof, borate compounds, barbituric acid compounds, triazine compounds, copper compounds, tin compounds, vanadium compounds, halogen compounds, aldehydes, thiol compounds, sulfites, bisulfites, and thiourea compounds include those mentioned in WO2008/087977.

The polymerization accelerator (e) may be contained alone, or two or more thereof may be contained in combination. The content of the polymerization accelerator (e) used in the present invention is not particularly limited. However, in view of the curability and other properties of the self-adhesive dental composite resin (X) obtained, the content of polymerization accelerator (e) is preferably 0.001 to 30 parts by mass, more preferably 0.01 to 10 parts by mass, even more preferably 0.1 to 5 parts by mass relative to total 100 parts by mass of monomers in the self-adhesive dental composite resin (X). When the content of polymerization accelerator (e) is at or above these lower limits, it is easier to provide a sufficient bond strength by allowing polymerization to sufficiently proceed. In this respect, the content of polymerization accelerator (e) is more preferably 0.05 parts or more by mass. When the content of polymerization accelerator (e) is at or below the foregoing upper limits, it is easier to provide sufficient adhesive properties, and to reduce precipitation of the polymerization accelerator (e) itself from the self-adhesive dental composite resin (X). In this respect, the content of polymerization accelerator (e) is more preferably 20 parts or less by mass.

### <Fluorine-Ion Releasing Substance>

A self-adhesive dental composite resin (X) of the present invention may additionally comprise a fluorine-ion releasing substance. By containing a fluorine-ion releasing substance, the self-adhesive dental composite resin (X) produced can impart acid resistance to tooth structure. Examples of the fluorine-ion releasing substance include metal fluorides such as sodium fluoride, potassium fluoride, sodium monofluorophosphate, lithium fluoride, and ytterbium fluoride. The fluorine-ion releasing substance may be contained alone, or two or more thereof may be contained in combination.

A self-adhesive dental composite resin (X) of the present invention may also comprise a known additive, provided that such additives do not cause a performance drop. Examples of additives that may be contained in the self-adhesive dental composite resin (X) include polymerization inhibitors, antioxidants, pigments, dyes, ultraviolet absorbers, solvents such as organic solvents, and thickeners. The additives may be used alone, or two or more thereof may be used in combination. In certain embodiments, the content of the solvent (for example, water, organic solvent) in the self-adhesive dental composite resin (X) is preferably less than 1 mass%, more preferably less than 0.1 mass%, even more preferably less than 0.01 mass% based on the total mass of the self-adhesive dental composite resin (X). In view of producing the effects of the present invention, it is preferable in another embodiment that the self-adhesive dental composite resin (X) be essentially free of water. The self-adhesive dental composite resin (X) being essentially free of water means that the water content is preferably 1 mass% or less, more preferably 0.1 mass% or less, even more preferably 0.01 mass% or less relative to the total amount of self-adhesive dental composite resin (X). The water content of the self-adhesive dental composite resin (X) may be 0 mass%.

Examples of the polymerization inhibitors include hydroquinone, hydroquinone monomethyl ether, dibutylhydroquinone, dibutylhydroquinone monomethyl ether, t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 1.0 parts by mass relative to total 100 parts by mass of monomers in the self-adhesive dental composite resin (X).

Examples of the fractions of the components in the self-adhesive dental composite resin (X) of a dental filling kit of the present invention are as follows. By taking the total amount of monomers as 100 parts by mass, it is preferable to comprise 1 to 40 parts by mass of monomer (a) having an acidic group, and 60 to 99 parts by mass of monomer (b) having no acidic group in total 100 parts by mass of the monomers, and 0.05 to 10 parts by mass of photopolymerization initiator (c-1), 100 to 900 parts by mass of filler (d), and 0.001 to 30 parts by mass of polymerization accelerator (e) relative to total 100 parts by mass of the monomers. More preferably, it is preferable to comprise 2.5 to 35 parts by mass of monomer (a) having an acidic group, and 65 to 97.5 parts by mass of monomer (b) having no acidic group in total 100 parts by mass of the monomers, and 0.1 to 5 parts by mass of photopolymerization initiator (c-1), 120 to 560 parts by mass of filler (d), and 0.01 to 10 parts by mass of polymerization accelerator (e) relative to total 100 parts by mass of the monomers. Even more preferably, it is preferable to comprise 5 to 30 parts by mass of monomer (a) having an acidic group, and 70 to 95 parts by mass of monomer (b) having no acidic group in total 100 parts by mass of the monomers, and 0.15 to 2.5 parts by mass of photopolymerization initiator (c-1), 150 to 400 parts by mass of filler (d), and 0.1 to 5 parts by mass of polymerization accelerator (e) relative to total 100 parts by mass of the monomers.

The self-adhesive dental composite resin (X) has the effect of alleviating the polymerization shrinkage stress that occurs when curing the bulk-fill dental composite resin (Y) under light, improving the cavity sealing properties immediately after application, and absorbing the impact from occlusion or other forces within the oral cavity. In view of this, it is preferable that the cured product of self-adhesive dental composite resin (X) after photocure have a flexural modulus of 1.5 to 7 GPa, more preferably 2 to 6.5 GPa, even more preferably 3 to 6 GPa. The measurement method of the flexural modulus of the cured product is as described in the EXAMPLES section below. The flexural modulus of the cured product can be set as the desired mechanical strength of the cured product by adjusting the type and/or content of each component in the self-adhesive dental composite resin (X).

By being used as a sealing material to seal cavities before filling a dental composite resin (Y) of the present invention, the self-adhesive dental composite resin (X) contained in a dental filling kit of the present invention can alleviate the polymerization shrinkage stress, and absorb the impact from occlusion or other forces within the oral cavity, as noted above. When used with the dental composite resin (Y) for bulk filling of cavities deeper than 2 mm, which normally require layered filling, the self-adhesive dental composite resin (X) can exhibit good cavity sealing properties even when the cured product is subjected to repeated loads of impact from occlusion or other forces within the oral cavity.

Overall, the self-adhesive dental composite resin (X) contained in a dental filling kit of the present invention with the foregoing components can alleviate the polymerization shrinkage stress, enabling improvement of the cavity sealing properties immediately after application, and absorption of impact from occlusion or other forces within the oral cavity. The self-adhesive dental composite resin (X), in combination with the dental composite resin (Y), can therefore exhibit good cavity sealing properties even when the cured product is subjected to repeated loads.

The following describes the components used in a dental composite resin (Y) of the present invention.

The dental composite resin (Y) comprises a monomer (b) having no acidic group, a polymerization initiator (c), and a filler (d), but does not comprise a monomer (a) having an acidic group. The monomer (a) having an acidic group is as described in conjunction with the self-adhesive dental composite resin (X). The dental composite resin (Y) is a bulk-fill composite resin, and has depth of cure of 4 mm or more. The dental composite resin (Y), in combination with the self-adhesive dental composite resin (X), exhibits good cavity sealing properties even when cavities deeper than 4 mm are filled in bulk, and shows good cavity sealing properties even when the cured product is subjected to repeated loads of impact from occlusion or other forces within the oral cavity.

Depth of cure of dental composite resin (Y) can be controlled by the transparency before and after cure, the curability of the polymerizable monomer, the type or content of polymerization initiator (c), and the type or content of filler (d). The transparency before and after cure can be appropriately adjusted mainly by adjusting the refractive indices of the polymerizable monomer and filler (d). As a rule, the depth of cure in photocure (depth of cure) tends to increase with increase of transparency before and after cure. The dental composite resin (Y) having depth of cure of 4 mm or more can be prepared by appropriately selecting the polymerizable monomer, polymerization initiator (c), and filler (d), and adjusting the content of these components. Here, depth of cure refers to a measured value in compliance with ISO 4049:2009.

### <Monomer (b) Having no Acidic Group>

The monomer (b) having no acidic group used in the dental composite resin (Y) is not particularly limited, and may be a known monomer, provided that it is a monomer (b) having no acidic group, different from the monomer (a) having an acidic group described in conjunction with the self-adhesive dental composite resin (X). Preferred for use as a monomer (b) having no acidic group are, for example, radical polymerizable monomers. Specific examples of radical polymerizable monomers include esters of acids such as α-cyanoacrylic acid, (meth)acrylic acid, α-halogenated acrylic acid, crotonic acid, cinnamic acid, sorbic acid, maleic acid, and itaconic acid; (meth)acrylamide and its derivatives; vinyl esters; vinyl ethers; mono-N-vinyl derivatives; and styrene derivatives. Preferred among these are (meth)acrylic acid esters.

Examples of the monomer (b) having no acidic group include a (meth)acrylic acid ester (b-4) having an aromatic ring but having no hydroxyl group, a (meth)acrylic acid ester (b-5) having an aromatic ring and a hydroxyl group, and a (meth)acrylic acid ester (b-6) having no aromatic ring and no hydroxyl group (hereinafter, these are also referred to as monomer (b-4), monomer (b-5), and monomer (b-6), respectively).

The (meth)acrylic acid ester (b-4) having an aromatic ring but having no hydroxyl group is not particularly limited, as long as it is a (meth)acrylic acid ester having an aromatic ring but having no hydroxyl group. The (meth)acrylic acid ester (b-4) having an aromatic ring but having no hydroxyl group has at least one aromatic ring. Examples of such compounds include (poly)ethoxylated bisphenol A di(meth)acrylates represented by the following general formula (4).

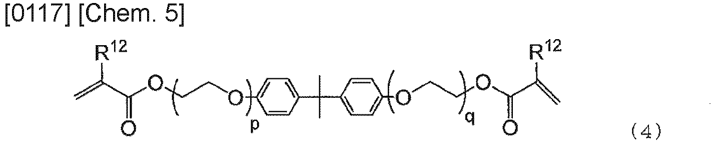

In the formula, p and q are 0 or positive numbers representing the average number of moles of ethoxy group added, and the sum of p and q is preferably 1 to 6, more preferably 2 to 4. R¹² each independently represents a hydrogen atom or a methyl group.

Specific examples of compounds represented by general formula (4) include 2,2-bis[4-(meth)acryloyloxypolyethoxyphenyl]propane (p + q = 2.6; hereinafter, also referred to as D-2.6E), 2,2-bis[4-(meth)acryloyloxypolyethoxyphenyl]propane (p + q = 6), 2,2-bis[4-(meth)acryloyloxydiethoxyphenyl]propane (p + q = 2), 2,2-bis[4-(meth)acryloyloxytetraethoxyphenyl]propane (p + q = 4), and 2,2-bis[4-(meth)acryloyloxypentaethoxyphenyl]propane (p + q = 5). Other examples include 2,2-bis[(meth)acryloyloxyphenyl]propane, 2,2-bis[4-(meth)acryloyloxydipropoxyphenyl]propane, 2-[4-(meth)acryloyloxydiethoxyphenyl]-2-[4-(meth)acryloyloxytriethoxyphenyl]propane, 2-[4-(meth)acryloyloxydipropoxyphenyl]-2-[4-(meth)acryloyloxytriethoxyphenyl]propane, 2,2-bis[4-(meth)acryloyloxypropoxyphenyl]propane, 2,2-bis[4-(meth)acryloyloxyisopropoxyphenyl]propane, and 2,2-bis[4-[3-(meth)acryloyloxy-2-(meth)acryloyloxypropoxy]phenyl]propane.

The (meth)acrylic acid ester (b-5) having an aromatic ring and a hydroxyl group is not particularly limited, as long as it is a (meth)acrylic acid ester having an aromatic ring and a hydroxyl group. The number of aromatic rings and the number of hydroxyl groups are independent of each other, and the (meth)acrylic acid ester (b-5) having an aromatic ring and a hydroxyl group has at least one aromatic group and at least one hydroxyl group. Examples of such compounds include 2,2-bis[4-[3-(meth)acryloyloxy-2-hydroxypropoxy]phenyl]propane, 2-[4-[3-(meth)acryloyloxy-2-hydroxypropoxy]phenyl]-2-[4-[2,3-di(meth)acryloyloxypropoxy]phenyl]propane, 2-[4-[3-(meth)acryloyloxy-2-hydroxypropoxy]phenyl]-2-[4-(meth)acryloyloxydiethoxyphenyl]propane, 2-[4-[3-(meth)acryloyloxy-2-hydroxypropoxy]phenyl]-2-[4-(meth)acryloyloxytriethoxyphenyl]propane, and 2-[4-[3-(meth)acryloyloxy-2-hydroxypropoxy]phenyl]-2-[4-(meth)acryloyloxydipropoxyphenyl]propane.

Examples of the (meth)acrylic acid ester (b-6) having no aromatic ring and no hydroxyl group include ethylene glycol di(meth)acrylate, triethylene glycol di(meth)acrylate, propylene glycol di(meth)acrylate, neopentyl glycol di(meth)acrylate, 1,6-hexanediol di(meth)acrylate, 1,10-decanediol di(meth)acrylate, methyl (meth)acrylate, isobutyl (meth)acrylate, benzyl (meth)acrylate, lauryl (meth)acrylate, 2-(N,N-dimethylamino)ethyl (meth)acrylate, 2,3-dibromopropyl (meth)acrylate, N,N'-(2,2,4-trimethylhexamethylene)bis[2-(aminocarboxy)propane-1,3-diol]tetramethacrylate, (meth)acryloyloxydodecylpyridinium bromide, (meth)acryloyloxydodecylpyridinium chloride, trimethylolpropane tri(meth)acrylate, trimethylolethane tri(meth)acrylate, pentaerythritol tetra(meth)acrylate, and N,N'-(2,2,4-trimethylhexamethylene)bis[2-(aminocarboxy)ethan-1-ol]dimethacrylate, a member of urethane dimethacrylates.

The monomer (b) having no acidic group may be used alone, or two or more thereof may be used in combination. In view of adhesive properties to self-adhesive dental composite resin (X) and mechanical strength, the monomer (b) having no acidic group is preferably a (meth)acrylic acid ester (b-4) having an aromatic ring but having no hydroxyl group, and/or a (meth)acrylic acid ester (b-5) having an aromatic ring and a hydroxyl group. When the total amount of the monomer (b) having no acidic group in dental composite resin (Y) is taken as 100 parts by mass, it is preferable that the total amount of (meth)acrylic acid ester (b-4) having an aromatic ring but having no hydroxyl group and/or (meth)acrylic acid ester (b-5) having an aromatic ring and a hydroxyl group be 20 parts or more by mass, more preferably 35 parts or more by mass, even more preferably 50 parts or more by mass. The monomer (b) having no acidic group used in the dental composite resin (Y) may comprise a component identical to the monomer (b) having no acidic group used in the self-adhesive dental composite resin (X), and may be an asymmetrical acrylamide methacrylic acid ester compound (b-1), a hydrophobic monomer (b-2), or a hydrophilic monomer (b-3) having no acidic group. Concerning the monomer (b) having no acidic group used in the dental composite resin (Y), the monomer (b) having no acidic group used in dental composite resin (Y) (for example, the monomer (b-4), (b-5), or (b-6)) may overlap the monomer (b) having no acidic group used in the self-adhesive dental composite resin (X) (for example, the compound (b-1), monomer (b-2), or monomer (b-3)), and, if necessary, these can be distinguished from each other by excluding one from the other (overlapping parts or all).

The refractive index of the monomer (b) having no acidic group can be adjusted by, for example, the ratio of the (meth)acrylic acid ester (b-4) having an aromatic ring but having no hydroxyl group and the (meth)acrylic acid ester (b-6) having no aromatic ring and no hydroxyl group. By appropriately adjusting the ratio of (b-4) and (b-6) according to the refractive index of the filler (d) used, it is possible to ensure transparency in the dental composite resin (Y) before and after cure, and achieve depth of cure of 4 mm or more.

The preferred combinations of monomers (b) having no acidic group include a combination of urethane dimethacrylate, a (poly)ethoxylated bisphenol A di(meth)acrylate represented by general formula (4), and triethylene glycol dimethacrylate, and a combination of urethane dimethacrylate, a (poly)ethoxylated bisphenol A di(meth)acrylate represented by general formula (4), and 1,10-decanediol dimethacrylate, more preferably a combination of urethane dimethacrylate, D-2.6E, and triethylene glycol dimethacrylate.

The monomer (b) having no acidic group has a viscosity at 23°C of preferably 1,500 cP or less, more preferably 200 to 1,000 cP, even more preferably 300 to 600 cP. The paste stickiness may increase when the viscosity is too high, whereas the formability may decrease when the viscosity is too low. Here, the viscosity refers to the viscosity of a polymerizable monomer-containing composition when two or more monomers (b) having no acidic group are used in combination. The viscosity can be measured using a known viscometer such as a B-type rotational viscometer or a cone-plate rotational viscometer, depending on the expected viscosity.

### <Polymerization Initiator (c)>

The polymerization initiator (c) can be selected from polymerization initiators that are commonly available. Particularly preferred for use are polymerization initiators used in dentistry. Among such polymerization initiators, a photopolymerization initiator (c-1) or a chemical polymerization initiator (c-2) may be used alone, or two or more thereof may be used in an appropriate combination.

Examples of the photopolymerization initiator (c-1) include (bis)acylphosphine oxides and salts thereof, thioxanthones or quaternary ammonium salts of thioxanthones, ketals, α-diketones, benzoin alkyl ether compounds, and α-aminoketone compounds. The photopolymerization initiator (c-1) used in the dental composite resin (Y) may be the same photopolymerization initiator (c-1) used in the self-adhesive dental composite resin (X).

Examples of the (bis)acylphosphine oxides and salts thereof include acylphosphine oxides and salts thereof, and bisacylphosphine oxides and salts thereof. Examples of the acylphosphine oxides and salts thereof include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, and salts of these (such as sodium salts and lithium salts, for example, sodium salts of 2,4,6-trimethylbenzoylphenylphosphine oxide, potassium salts of 2,4,6-trimethylbenzoyldiphenylphosphine oxide, and ammonium salts of 2,4,6-trimethylbenzoyldiphenylphosphine oxide). Examples of the bisacylphosphine oxides and salts thereof include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphine oxide, and salts of these (such as sodium salts and lithium salts).

Preferred among these (bis)acylphosphine oxides and salts thereof are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the thioxanthones or quaternary ammonium salts of thioxanthones include thioxanthone, 2-chlorothioxanthen-9-one, 2-hydroxy-3-(9-oxy-9H-thioxanthen-4-yloxy)-N,N,N-trimethyl-1-propaneaminium chloride, 2-hydroxy-3-(1-methyl-9-oxy-9H-thioxanthen-4-yloxy)-N,N,N-trimethyl-1-propaneaminium chloride, 2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N,N,N-trimethyl-1-propaneaminium chloride, 2-hydroxy-3-(3,4-dimethyl-9H-thioxanthen-2-yloxy)-N,N,N-trimethyl-1-propaneam inium chloride, and 2-hydroxy-3-(1,3,4-trimethyl-9-oxo-9H-thioxanthen-2-yloxy)-N,N,N-trimethyl-1-propaneaminium chloride.

Among these thioxanthones or quaternary ammonium salts of thioxanthones, the preferred thioxanthone is 2-chlorothioxanthen-9-one, and the preferred quaternary ammonium salt of thioxanthones is 2-hydroxy-3-(3,4-dimethyl-9H-thioxanthen-2-yloxy)-N,N,N-trimethyl-1-propaneaminium chloride.

Examples of the ketals include benzyl dimethyl ketal, and benzyl diethyl ketal.

Examples of the α-diketones include diacetyl, benzyl, camphorquinone, 2,3-pentadione, 2,3-octadione, 9,10-phenanthrenequinone, 4,4'-oxybenzyl, and acenaphthenequinone. In view of the maximum absorption wavelength occurring in the visible light region, preferred is camphorquinone.

Examples of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

Preferred for use among these photopolymerization initiators (c-1) is at least one selected from the group consisting of a (bis)acylphosphine oxide or a salt thereof, and an α-diketone.

Preferred for use as chemical polymerization initiator (c-2) are azo compounds and organic peroxides. The azo compounds and organic peroxides are not particularly limited, and may be known compounds. Typical examples of azo compounds include 2,2'-azobis(isobutyronitrile), 2,2'-azobis(4-methoxy-2,4-dimethylvaleronitrile), and 2,2'-azobis(2,4-dimethylvaleronitrile). Typical examples of organic peroxides include ketone peroxides, hydroperoxides, diacyl peroxides, dialkyl peroxides, peroxyketals, peroxyesters, and peroxydicarbonates. The chemical polymerization initiator (c-2) used in the dental composite resin (Y) may be the same chemical polymerization initiator (c-2) used in the self-adhesive dental composite resin (X).

Examples of the ketone peroxides include methyl ethyl ketone peroxide, methyl isobutyl ketone peroxide, methylcyclohexanone peroxide, and cyclohexanone peroxide.

Examples of the hydroperoxides include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butyl cumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and 4,4-bis(t-butylperoxy)valeric acid-n-butyl ester.

Examples of the peroxyesters include α-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydroterephthalate, t-butyl peroxy-3,3,5-trimethylhexanoate, t-butyl peroxyacetate, t-butyl peroxybenzoate, and t-butyl peroxymaleic acid.

Examples of the peroxydicarbonates include di(3-methoxybutyl)peroxydicarbonate, di(2-ethylhexyl)peroxydicarbonate, bis(4-t-butylcyclohexyl)peroxydicarbonate, diisopropylperoxydicarbonate, di-n-propylperoxydicarbonate, di(2-ethoxyethyl)peroxydicarbonate, and diallyl peroxydicarbonate.

From an overall balance of safety, storage stability, and radical generating potential, preferred for use among these organic peroxides are diacyl peroxides, more preferably benzoyl peroxide.

In view of depth of cure of dental composite resin (Y), the content of polymerization initiator (c) is preferably 0.01 to 10 parts by mass, more preferably 0.1 to 7 parts by mass, even more preferably 0.15 to 6 parts by mass, particularly preferably 0.5 to 5 parts by mass relative to total 100 parts by mass of polymerizable monomers in the dental composite resin (Y). When the content of polymerization initiator (c) is at or above the foregoing lower limits, it is easier to achieve depth of cure of 4 mm or more in the dental composite resin (Y). When the content of polymerization initiator (c) is at or below the foregoing upper limits, the polymerization initiator (c) helps reduce an increase of polymerization shrinkage stress during polymerization of dental composite resin (Y), making it easier to provide excellent cavity sealing properties.

### <Filler (d)>

It is preferable that a dental composite resin (Y) of the present invention comprise a filler (d), in order to adjust the ease of handling, and to increase the mechanical strength of the cured product. Examples of such fillers include an inorganic filler (d-1), an organic-inorganic composite filler (d-2), and an organic filler (d-3). A certain preferred embodiment is, for example, a dental filling kit in which the filler (d) contained in the dental composite resin (Y) comprises an inorganic filler (d-1).

The material or type of inorganic filler (d-1) is not particularly limited, as long as it does not hinder the effectiveness of the present invention. The inorganic filler (d-1) may be a known inorganic filler used in applications such as dental restoration material compositions. Examples include various types of glass, and particle aggregates.

Examples of the inorganic filler (d-1) include various types of glass (those containing silica as a main component, and, optionally, oxides of elements such as heavy metals, boron, zirconium, titanium, and aluminum), including, for example, glass powders of common compositions, such as fused silica, soda-lime-silica glass, E glass, C glass, and borosilicate glass (PYREX^{®} glass); and dental glass powders such as strontium boroaluminosilicate glass, barium glass (e.g., barium silicate glass E-2000, E-3000, manufactured by ESSTECH, barium boroaluminosilicate glass GM27884, 8235 series (e.g., 8235 UF0.7), manufactured by Schott), lanthanum glass GM31684 (manufactured by Schott), and fluoroaluminosilicate glass GM35429, G018-091, G018-117 (manufactured by Schott). Other examples of inorganic filler (d-1) include various ceramics, composite oxides (for example, silica-titania-barium oxide, silica-zirconia, silica-titania, silica-alumina, silica-alumina-zirconia), diatomaceous earth, kaolin, clay minerals (such as montmorillonite), activated earth, synthetic zeolite, mica, quartz, silica, calcium fluoride, ytterbium fluoride, yttrium fluoride, calcium phosphate, barium sulfate, zirconium dioxide, titanium dioxide, and hydroxyapatite. In view of refractive index, preferred are barium glass, silica-zirconia composite oxide, silica-titania composite oxide, silica-alumina-zirconia composite oxide, quartz, and ytterbium fluoride.

The inorganic filler (d-1) may be used alone, or two or more thereof may be used in combination. In order to adjust the flowability of the dental composite resin (Y), the inorganic filler (d-1) may be used after an optional surface treatment with a known surface treatment agent such as a silane coupling agent. The surface treatment agent and surface treatment method may be the same surface treatment agent or surface treatment method used for the inorganic filler (d-1) of the self-adhesive dental composite resin (X). The shape of inorganic filler is not particularly limited, and is preferably spherical, near spherical, or irregular. Here, the term "near spherical" means particles that appear round in shape when observed in a unit field of a scanning electron micrograph (hereinafter, referred to with the abbreviation "SEM"), and that have an average uniformity of 0.6 or more as calculated by dividing the diameter of a particle perpendicular to its maximum diameter by the maximum diameter. In view of improving the mechanical strength of a cured product of the dental composite resin (Y), the inorganic filler (d-1) used is preferably an irregularly shaped filler.

In view of the workability of dental composite resin (Y) and the polishability of the cured product, the inorganic filler (d-1) has an average particle diameter of 0.001 to 50 µm, preferably 0.005 to 30 µm, more preferably 0.01 to 20 µm. When the average particle diameter is at or above these lower limits, it is possible to reduce a decrease of workability due to increased stickiness of dental composite resin (Y). When the average particle diameter is at or below the foregoing upper limits, the cured product can have the desired polishability. The method of measurement of average particle diameter is as described in the EXAMPLES section below.

The inorganic filler (d-1) may be an aggregate of particles (aggregate filler) prepared by aggregating inorganic ultrafine particles or various types of glass such as above. The preparation method of such aggregated particles is not particularly limited, and known methods may be used. For example, when preparing an aggregate of particles using inorganic ultrafine particles as a raw material, the preferred method of preparing a strong aggregate of particles from commercially available inorganic ultrafine particles heats the inorganic ultrafine particles to a temperature range just below the temperature that melts the inorganic ultrafine particles so that adjoining inorganic ultrafine particles lightly fuse together and increase cohesion. Here, the inorganic ultrafine particles may have a form of an aggregate before heating, in order to control the shape of the particle aggregate. Such an aggregate can be formed, for example, by applying pressure to the inorganic ultrafine particles placed in a suitable container, or by dispersing the inorganic ultrafine particles in a solvent, and removing the solvent using a method such as spray drying. When the average particle diameter of inorganic filler (d-1) ranges from 0.7 to 2.0 µm, it is preferable to contain inorganic ultrafine particles as inorganic filler (d-1) because it helps reduce separation of the polymerizable monomers and hardening of the paste due to the repetitive application of high pressure to the paste that comes into contact with the seal when the paste is filled into a syringe and collected through the seal by pressing the plunger, even when the inorganic filler (d-1) uses inorganic ultrafine particles as a raw material, and has a form of a particle aggregate.

The inorganic ultrafine particles have an average particle diameter of preferably 5 to 50 nm, more preferably 10 to 40 nm. The average particle diameter of inorganic ultrafine particles can be measured as a mean value of the particle diameters of randomly selected 100 ultrafine particles in an electron micrograph of inorganic ultrafine particles. When the inorganic ultrafine particles are non-spherical, the particle diameter is determined as an arithmetic mean value of the maximum and minimum lengths of the inorganic ultrafine particles.

The inorganic ultrafine particles may be any known inorganic ultrafine particles. Preferred examples include particles of inorganic oxides such as silica, alumina, titania, and zirconia, or particles of composite oxides of these, along with calcium phosphate, hydroxyapatite, yttrium fluoride, and ytterbium fluoride. Preferably, the inorganic ultrafine particles are, for example, silica, alumina, or titania particles prepared by flame hydrolysis. Examples of such particles include Aerosil, Aeroxide AluC, and Aeroxide TiO₂ P25 manufactured by Nippon Aerosil Co., Ltd. under these trade names, and VP Zirconium Oxide 3-YSZ, and VP Zirconiumxide 3-YSZ PH manufactured by EVONIK.

The filler (d) used for the dental composite resin (Y) may be an organic-inorganic composite filler (d-2). The filler (d) used for the dental composite resin (Y) may be solely an organic-inorganic composite filler (d-2), or may comprise an inorganic filler (d-1) and an organic-inorganic composite filler (d-2).

The organic-inorganic composite filler (d-2) has an average particle diameter of preferably 3 to 25 µm, more preferably 4 to 20 µm, even more preferably 5 to 20 µm. When the average particle diameter of organic-inorganic composite filler (d-2) is at or above these lower limits, it is possible to reduce a decrease of workability due to increased stickiness of dental composite resin (Y). When the average particle diameter is at or below the foregoing upper limits, it is easier to inhibit property changes such as changes in the roughness or dryness of a paste of dental composite resin (Y), providing good workability.

The organic-inorganic composite filler (d-2) may be used alone, or two or more thereof may be used in combination. In view of the workability of a dental composite resin (Y) of the present invention in a paste form before cure, it is preferable to use two or more organic-inorganic composite fillers (d-2) of different average particle diameters in combination. In certain embodiments, the organic-inorganic composite filler (d-2) preferably comprises an organic-inorganic composite filler (d-2a) having an average particle diameter of 13 µm or more and 25 µm or less. In another embodiment, the organic-inorganic composite filler (d-2) preferably comprises an organic-inorganic composite filler (d-2b) having an average particle diameter of 3 µm or more and 10 µm or less. In yet another embodiment, the organic-inorganic composite filler (d-2) preferably comprises an organic-inorganic composite filler (d-2a) having an average particle diameter of 13 µm or more and 25 µm or less, and an organic-inorganic composite filler (d-2b) having an average particle diameter of 3 µm or more and 10 µm or less. The organic-inorganic composite filler (d-2a) may have an average particle diameter of 15 µm or more and 20 µm or less. The organic-inorganic composite filler (d-2b) may have an average particle diameter of 4 µm or more and 8 µm or less.

The method of production of organic-inorganic composite filler (d-2) is not particularly limited. For example, the organic-inorganic composite filler (d-2) can be prepared by adding a known polymerizable monomer and a known polymerization initiator to a known inorganic filler (d-1), polymerizing the filler mixture in paste form by solution polymerization, suspension polymerization, emulsion polymerization, or bulk polymerization, and pulverizing the resulting polymer.

The polymerizable monomer used for the production of organic-inorganic composite filler (d-2) is not particularly limited, and may be the same polymerizable monomer exemplified as the monomer (b) having no acidic group of dental composite resin (Y), or a polymerizable monomer of the same composition as the monomer (b) having no acidic group of dental composite resin (Y). Also preferred for use are polymerizable monomers that have been subjected to a purification process. Using a polymerizable monomer that has not undergone a purification process may result in the organic-inorganic composite filler (d-2) acquiring color due to impurities within the polymerizable monomer. This not only prevents making adjustments to achieve the desired shade but can lead to a decrease in the aesthetic quality of the dental composite resin (Y) after cure.

The polymerization initiator used for the production of organic-inorganic composite filler (d-2) is not particularly limited, and may be a known polymerization initiator. Examples include photopolymerization initiators that make use of, for example, ultraviolet light or visible light, and chemical polymerization initiators that employ, for example, a reaction between peroxides and accelerators, or heat. The polymerization initiator may be appropriately selected from the polymerization initiators exemplified as polymerization initiator (c), and may be the same or different from the polymerization initiator (c).

A certain preferred embodiment is, for example, a dental filling kit in which the filler (d) contained in the dental composite resin (Y) comprises an organic-inorganic composite filler (d-2), and the organic-inorganic composite filler (d-2) is an organic-inorganic composite filler using an inorganic filler (d-1a) having an average particle diameter of 0.5 µm or less (hereinafter, also referred to simply as "inorganic filler (d-1a)").

The inorganic filler (d-1a) has an average particle diameter of 0.5 µm or less, preferably 0.005 to 0.3 µm, more preferably 0.01 to 0.2 µm. When the inorganic filler (d-1a) used for the organic-inorganic composite filler (d-2) has an average particle diameter of 0.5 µm or less, the dental composite resin (Y) can exhibit good polishability after cure.

The content of the inorganic filler (d-1) (preferably, inorganic filler (d-1a)) contained in the organic-inorganic composite filler (d-2) is preferably 40 to 90 mass%, more preferably 45 to 85 mass%, even more preferably 55 to 85 mass% relative to the total mass of the organic-inorganic composite filler. With these contents, it is possible to control the mechanical strength of the cured dental composite resin (Y) to a desired value.

The material of the inorganic filler (d-1) (preferably, inorganic filler (d-1a)) used for the organic-inorganic composite filler (d-2) is not particularly limited, and the inorganic filler (d-1) may be any of the inorganic fillers exemplified as inorganic filler (d-1), or may be inorganic ultrafine particles.

In view of enhancing the affinity to the polymerizable monomers, or improving the mechanical strength of the organic-inorganic composite filler (d-2) by increasing the chemical bonding with the polymerizable monomers, the inorganic filler (d-1) used for the organic-inorganic composite filler (d-2) may be used after an optional surface treatment with a known surface treatment agent such as a silane coupling agent. The surface treatment agent and surface treatment method may be any of the agents or methods exemplified in conjunction with the inorganic filler (d-1).

The inorganic ultrafine particles used for the inorganic filler (d-1a) may be any known inorganic ultrafine particles, as with the case of inorganic filler (d-1). The preferred materials are also the same as in the inorganic filler (d-1).

The inorganic ultrafine particles used for the inorganic filler (d-1a) are also the same as inorganic filler (d-1) in terms of the preferred range of average particle diameters, and the measurement method of average particle diameter.

Because inorganic ultrafine particles are used for the organic-inorganic composite filler (d-2) with polymerizable monomers, it is preferable that the inorganic ultrafine particles be pre-treated with a surface treatment agent to enhance the affinity to the polymerizable monomers, and to improve the mechanical strength of the organic-inorganic composite filler (d-2) by increasing the chemical binding with the polymerizable monomers. The surface treatment agent and surface treatment method may be any of the agents or methods exemplified in conjunction with the inorganic filler (d-1).

Depending on intended use, the components of the organic-inorganic composite filler (d-2) may additionally include known additives such as polymerization inhibitors, pH adjusters, ultraviolet absorbers, antioxidants, antimicrobial agents, fluorescent agents, surface active agents, dispersants, and thickeners, provided that it does not hinder the effectiveness of the invention. These may be used alone, or two or more thereof may be used in combination. Examples of the polymerization inhibitors include 2,6-di-butylhydroxytoluene, hydroquinone, dibutylhydroquinone, dibutylhydroquinone monomethyl ether, and 2,6-t-butylphenol. These may be used alone, or two or more thereof may be used in combination. The ultraviolet absorbers may be known compounds, including, for example, triazine ultraviolet absorbers, benzotriazole ultraviolet absorbers, benzophenone ultraviolet absorbers, benzoate ultraviolet absorbers, and hindered amine light stabilizers. These may be used alone, or two or more thereof may be used in combination.

The content of the filler (d) in dental composite resin (Y) is preferably 160 to 600 parts by mass, more preferably 220 to 400 parts by mass, even more preferably 250 to 340 parts by mass relative to total 100 parts by mass of polymerizable monomers in the dental composite resin (Y). When the content of filler (d) is low, formability may decrease due to increased fluidity of a paste of dental composite resin (Y) before cure, whereas the paste may become excessively hard, and workability may decrease when the content is too high.

In view of the mechanical strength and depth of cure of the cured product, it is preferable in certain embodiments that the content of the filler (d) in dental composite resin (Y) be 55 to 95 parts by mass, more preferably 60 to 90 parts by mass, even more preferably 61 to 88 parts by mass in total 100 parts by mass of the dental composite resin (Y).

When the filler (d) in dental composite resin (Y) comprises an organic-inorganic composite filler (d-2), it is preferable to use the inorganic filler (d-1), in addition to the organic-inorganic composite filler (d-2). In this case, the content of the inorganic filler (d-1) is preferably 60 to 300 parts by mass, more preferably 70 to 150 parts by mass, even more preferably 80 to 120 parts by mass relative to total 100 parts by mass of polymerizable monomers in the dental composite resin (Y).

The content of the organic-inorganic composite filler (d-2) in dental composite resin (Y) is preferably 100 to 300 parts by mass, more preferably 150 to 250 parts by mass, even more preferably 170 to 220 parts by mass relative to total 100 parts by mass of polymerizable monomers in the dental composite resin (Y). Formability may decrease when the content of organic-inorganic composite filler (d-2) is excessively low, whereas the paste may become excessively hard, and workability may decrease when the content is too high. By containing the organic-inorganic composite filler (d-2) in the above mass ratios, the paste workability can more easily improve.

The mass ratio of the content of the inorganic filler (d-1) and the content of the organic-inorganic composite filler (d-2) in the dental composite resin (Y) is not particularly limited. It is, however, preferable that the organic-inorganic composite filler (d-2) be higher in content than the inorganic filler (d-1). That is, the mass ratio is preferably (d-2)/(d-1) > 1. With the organic-inorganic composite filler (d-2) being higher in content than the inorganic filler (d-1), the dental composite resin (Y) can have reduced stickiness and improved formability and polishability. In the present invention, the total content of inorganic filler (d-1) and organic-inorganic composite filler (d-2) may be 50 mass% or more, 60 mass% or more, or 70 mass% or more in the dental composite resin (Y). The total content of inorganic filler (d-1) and organic-inorganic composite filler (d-2) may be 83 mass% or less.

The dental composite resin (Y) may comprise fillers other than inorganic filler (d-1), organic-inorganic composite filler (d-2), and organic filler (d-3).

The refractive index of inorganic filler (d-1) is not particularly limited. However, the transparency and depth of cure of the dental composite resin (Y) before and after cure can be adjusted by, for example, adjusting the refractive indices of the inorganic filler (d-1) and polymerizable monomers.

The organic filler (d-3) used for the dental composite resin (Y) may be the same organic filler (d-3) used for the self-adhesive dental composite resin (X).

### <Polymerization Accelerator (e)>

The dental composite resin (Y) may additionally comprise a polymerization accelerator (e). Examples of the polymerization accelerator (e) include amines, sulfinic acid and salts thereof, aldehydes, and thiol compounds. The polymerization accelerator (e) may be used alone, or two or more thereof may be used in combination. The polymerization accelerator (e) used for the dental composite resin (Y) may be the same polymerization accelerator (e) used for the self-adhesive dental composite resin (X).

The amines can be categorized into aliphatic amines and aromatic amines. Examples of the aliphatic amines include the same aliphatic amines exemplified for the polymerization accelerator (e) used in the self-adhesive dental composite resin (X). In view of the curability and storage stability of dental composite resin (Y), preferred are tertiary aliphatic amines, more preferably 2-(dimethylamino)ethyl methacrylate, N-methyldiethanolamine, and triethanolamine.

Examples of the aromatic amines include the same aromatic amines exemplified for the polymerization accelerator (e) used in the self-adhesive dental composite resin (X). In view of the ability to improve the curability of dental composite resin (Y), preferred for use is at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Examples of the sulfinic acid and salts thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Preferred for use are sodium benzenesulfinate, sodium p-toluenesulfinate, and sodium 2,4,6-triisopropylbenzenesulfinate.

Examples of the aldehydes include terephthalaldehyde, and benzaldehyde derivatives. Examples of the benzaldehyde derivatives include dimethylaminobenzaldehyde, p-methoxybenzaldehyde, p-ethoxybenzaldehyde, and p-n-octyloxybenzaldehyde. In view of the ability to improve the curability of dental composite resin (Y), preferred for use is p-n-octyloxybenzaldehyde.

Examples of the thiol compounds include 3-mercaptopropyltrimethoxysilane, 2-mercaptobenzoxazole, decanethiol, and thiobenzoic acid.

The content of the polymerization accelerator (e) in dental composite resin (Y) is not particularly limited, and is preferably 0.01 to 10 parts by mass, more preferably 0.1 to 7 parts by mass, even more preferably 0.2 to 5 parts by mass relative to total 100 parts by mass of polymerizable monomers in the dental composite resin (Y). When the content of polymerization accelerator (e) is at or above these lower limits, it is easier to achieve depth of cure of 4 mm or more in the dental composite resin (Y). When the content of polymerization accelerator (e) is at or below the foregoing upper limits, the polymerization accelerator (e) helps reduce an increase of polymerization shrinkage stress during polymerization of dental composite resin (Y), making it easier to provide excellent cavity sealing properties.

Depending on intended use, the dental composite resin (Y) may additionally comprise components such as polymerization inhibitors, pH adjusters, ultraviolet absorbers, antioxidants, antimicrobial agents, fluorescent agents, surface active agents, and dispersants, provided that it does not hinder the effectiveness of the invention. The polymerization inhibitors and ultraviolet absorbers may be any of the polymerization inhibitors or ultraviolet absorbers exemplified as optional components of the organic-inorganic composite filler (d-2).

In certain embodiments, the content of the solvent (for example, water, organic solvent) in the dental composite resin (Y) is preferably less than 1 mass%, more preferably less than 0.1 mass%, even more preferably less than 0.01 mass% based on the total mass of the dental composite resin (Y). In view of the present invention exhibiting its effects, it is preferable in other embodiments that the dental composite resin (Y) be essentially free of water. In referring to dental composite resin (Y) being essentially free of water, the water content is preferably 1 mass% or less, more preferably 0.1 mass% or less, even more preferably 0.01 mass% or less, or may be 0 mass% relative to the total amount of the dental composite resin (Y).

In view of cavity sealing properties, it is preferable in a dental filling kit of the present invention that the flexural modulus ratio ((Y)/(X)) between a cured product of the self-adhesive dental composite resin (X) and a cured product of the dental composite resin (Y) range from 0.9 to 5.0, more preferably from 1.1 to 3.5, even more preferably from 1.2 to 3.0. In view of producing even superior cavity sealing properties after repeated loading, the flexural modulus ratio is particularly preferably 1.3 to 2.9. The flexural modulus ratio ((Y)/(X)) of cured products can be achieved by adjusting the type and/or content of each component in the self-adhesive dental composite resin (X) and dental composite resin (Y) to adjust the flexural moduli of the self-adhesive dental composite resin (X) and dental composite resin (Y) to desired values in their respective cured products. The flexural modulus ratio can be adjusted within the foregoing ranges by adjusting, for example, the type and/or content of polymerizable monomers, the type or content of polymerization initiator (c), or the type and/or content of filler (d). Another way of adjusting the flexural modulus ratio ((Y)/(X)) of cured products is to provide the same or higher content for the filler (d) contained in the dental composite resin (Y) than the filler (d) contained in the self-adhesive dental composite resin (X).

In certain embodiments, the cured product of dental composite resin (Y) after photocure has a flexural modulus of preferably 1.5 to 25 GPa, more preferably 3 to 20 GPa, even more preferably 5 to 18 GPa.

A certain embodiment is, for example, a cavity sealing method using a dental filling kit, wherein the dental filling kit comprises a self-adhesive dental composite resin (X) that comprises a monomer (a) having an acidic group, a monomer (b) having no acidic group, a polymerization initiator (c), and a filler (d); and a dental composite resin (Y) that comprises a monomer (b) having no acidic group, a polymerization initiator (c), and a filler (d), but does not comprise a monomer (a) having an acidic group, and the method comprises sealing a cavity with the self-adhesive dental composite resin (X), and filling the cavity with the dental composite resin (Y).

The cavity may be, for example, a cavity deeper than 2 mm.

The sealing method is not particularly limited, and sealing can be achieved using a known instrument such as a spatula. Considering the subsequent filling of dental composite resin (Y) after sealing, it is preferable to use a small amount of self-adhesive dental composite resin (X) for sealing. For example, the self-adhesive dental composite resin (X) may seal a depth of less than 1 mm in the cavity.

The present invention encompasses embodiments combining the foregoing features in various ways (for example, an embodiment combining the preferred content ranges of a certain component with the more preferred content ranges of some other component) within the technical idea of the present invention, provided that the present invention can exhibit the effects produced by combining the self-adhesive dental composite resin (X) and the dental composite resin (Y).

### EXAMPLES

The following describes the present invention in greater detail using Examples and Comparative Examples. However, the present invention is not limited to the following descriptions. In the following, "part(s)" means "part(s) by mass", unless otherwise specifically stated.

The components of the dental filling kits of Examples and Comparative Examples are presented below, along with the abbreviations.

### [Monomer (a) Having Acidic Group]

MDP: 10-methacryloyloxydecyl dihydrogen phosphate
GPDM: glycerol phosphate dimethacrylate

### [Monomer (b) Having no Acidic Group]

Bis-GMA: 2,2-bis[4-(2-hydroxy-3-methacryloyloxypropoxy)phenyl]propane
UDMA: 2,2,4-trimethylhexamethylenebis(2-carbamoyloxyethyl)dimethacrylate
D-2.6E: 2,2-bis(4-methacryloyloxypolyethoxyphenyl)propane (with an average of 2.6 moles of ethoxy group added)
3G: triethylene glycol dimethacrylate
MAEA: N-methacryloyloxyethylacrylamide
DD: 1,10-decanediol dimethacrylate

### [Photopolymerization Initiator (c-1)]

[Water-Soluble Photopolymerization Initiator (c-1a)]
   Li-TPO: Lithium phenyl(2,4,6-trimethylbenzoyl)phosphinate
[Water-Insoluble Photopolymerization Initiator (c-1b)]
   CQ: camphorquinone
   TPO: 2,4,6-trimethylbenzoyldiphenylphosphine oxide

### [Filler (d)]

### [Inorganic Filler (d-1)]

D-1: ultrafine particulate silica Aerosil^{®} R 972 manufactured by Nippon Aerosil Co., Ltd.; average particle diameter: 16 nm
D-2: A silica stone powder (silica manufactured by Nitchitsu Co., Ltd. under the trade name Hi-Silica) was pulverized with a ball mill to obtain a pulverized silica stone powder. The pulverized silica stone powder had an average particle diameter of 2.2 µm as measured by volume with a laser diffraction particle size distribution analyzer (Model SALD-2300, manufactured by Shimadzu Corporation). One-hundred parts by mass of the pulverized silica stone powder was surface treated with four parts by mass of γ-methacryloyloxypropyltrimethoxysilane by an ordinary method to obtain an inorganic filler (D-2) (hereinafter, also referred to simply as "D-2").
D-3: A barium silicate glass (product name E-3000, manufactured by ESSTECH) was pulverized with a ball mill to obtain a barium silicate glass powder. The barium silicate glass powder had an average particle diameter of 2.4 µm as measured by volume with a laser diffraction particle size distribution analyzer (Model SALD-2300, manufactured by Shimadzu Corporation). One-hundred parts by mass of the barium silicate glass powder was surface treated with three parts by mass of γ-methacryloyloxypropyltrimethoxysilane by an ordinary method to obtain an inorganic filler (D-3) (hereinafter, also referred to simply as "D-3").
D-4: A three-neck flask was charged with 100 g of barium boroaluminosilicate glass (8235 UF0.7 grade, manufactured by Schott; average particle diameter: 0.7 µm), 6 g of γ-methacryloyloxypropyltrimethoxysilane, and 200 mL of a 0.3 mass% aqueous solution of acetic acid, and these were stirred at room temperature for 2 hours. After removing water by freeze drying, a heat treatment was carried out at 80°C for 5 hours to obtain an inorganic filler (D-4) (hereinafter, also referred to simply as "D-4").
D-5: A three-neck flask was charged with 100 g of ultrafine particulate silica powder (Aerosil^{®} 130 manufactured by Nippon Aerosil Co., Ltd.; average particle diameter: 0.02 µm) prepared by flame hydrolysis, 20 g of γ-methacryloyloxypropyltrimethoxysilane, and 200 mL of a 0.3 mass% aqueous solution of acetic acid, and these were stirred at room temperature for 2 hours. After removing water by freeze drying, a heat treatment was carried out at 80°C for 5 hours to obtain an inorganic filler (D-5) (hereinafter, also referred to simply as "D-5").
D-6: A three-neck flask was charged with 100 g of ultrafine particulate silica powder (Aerosil^{®} OX 50 manufactured by Nippon Aerosil Co., Ltd.; average particle diameter: 0.04 µm) prepared by flame hydrolysis, 7 g of γ-methacryloyloxypropyltrimethoxysilane, and 200 mL of a 0.3 mass% aqueous solution of acetic acid, and these were stirred at room temperature for 2 hours. After removing water by freeze drying, a heat treatment was carried out at 80°C for 5 hours to obtain an inorganic filler (D-6) (hereinafter, also referred to simply as "D-6").
D-7: A three-neck flask was charged with 100 g of barium boroaluminosilicate glass (GM27884 NF180 grade, manufactured by Schott; average particle diameter: 0.18 µm), 13 g of γ-methacryloyloxypropyltrimethoxysilane, and 200 mL of a 0.3 mass% aqueous solution of acetic acid, and these were stirred at room temperature for 2 hours. After removing water by freeze drying, a heat treatment was carried out at 80°C for 5 hours to obtain an inorganic filler (D-7) (hereinafter, also referred to simply as "D-7").
D-8: A three-neck flask was charged with 100 g of barium boroaluminosilicate glass (GM27884 UF1.0 grade, manufactured by Schott; average particle diameter: 1.0 µm), 6 g of γ-methacryloyloxypropyltrimethoxysilane, and 200 mL of a 0.3 mass% aqueous solution of acetic acid, and these were stirred at room temperature for 2 hours. After removing water by freeze drying, a heat treatment was carried out at 80°C for 5 hours to obtain an inorganic filler (D-8) (hereinafter, also referred to simply as "D-8").
D-9: A barium boroaluminosilicate glass (8235 K4 manufactured by Schott) was pulverized with a ball mill to obtain a barium boroaluminosilicate glass powder. The glass powder had an average particle diameter of about 2.4 µm as measured with a laser diffraction particle size distribution analyzer (Model SALD-2300, manufactured by Shimadzu Corporation). One-hundred parts by mass of the glass powder was surface treated with three parts by mass of γ-methacryloyloxypropyltrimethoxysilane by an ordinary method to obtain an inorganic filler (D-9) (hereinafter, also referred to simply as "D-9").

### [Organic-Inorganic Composite Filler (d-2)]

D-10: One-hundred parts by mass of D-6 was added as inorganic filler (d-1) (inorganic filler (d-1) content: 60 mass%) to one-hundred parts by mass of a composition containing 1 mass% of pre-dissolved AIBN (polymerization initiator (c)) along with polymerizable monomers as per the mass proportions shown Table 1. After mixing these into a paste, the paste was subjected to 5 hours of thermal polymerization at 100°C in a reduced pressure atmosphere. The resulting cured product of polymerization was pulverized to a desired average particle diameter with a vibration ball mill. The pulverized filler (100 g) was surface treated by 90°C, 5-hour refluxing with a 200 mL ethanol solution containing 2 mass% of γ-methacryloyloxypropyltrimethoxysilane to obtain an organic-inorganic composite filler (D-10).

D-11: The organic-inorganic composite filler (D-11) shown in Table 1 was prepared in the same manner as the organic-inorganic composite filler (D-10), except that the content and the average particle diameter of the inorganic filler were varied to the desired values with the use of the inorganic filler (d-1) and polymerizable monomers shown in Table 1.

### [Polymerization Accelerator (e)]

### DABE: ethyl 4-(N,N-dimethylamino)benzoate (polymerization accelerator for photopolymerization initiator)

### [Other]

### BHT: 2,6-di-t-butyl-4-methylphenol (stabilizer)

**[Table 1]**

| | Inorganic filler (d-1) | | Polymerizable monomer¹⁾ | | | | Organic-inorganic composite filler (d-2) | |
|---|---|---|---|---|---|---|---|---|
| | Type | Average particle diameter (µm) | UDMA | Bis-GMA | 3G | DD | Content of (d-1) (mass%) | Average particle diameter (µm) |
| D-10 | D-6 | 0.04 | 70 | - | - | 30 | 60 | 15 |
| D-11 | D-7 | 0.18 | - | 50 | 50 | - | 80 | 5 |

| | | | | | | | | |
|---|---|---|---|---|---|---|---|---|
| 1)Content of each monomer in total amount of polymerizable monomers (mass%) | | | | | | | | |

### [Examples 1 to 9]

The raw materials of self-adhesive dental composite resin (X) shown in Table 2, and the raw materials of dental composite resin (Y) shown in Table 3 were separately mixed and kneaded at ordinary temperature (23°C) in the dark to prepare a paste-like self-adhesive dental composite resin (X) (compositions 1 to 6) and a paste-like bulk-fill dental composite resin (Y) (compositions 7 to 9). These were evaluated for their cavity sealing properties after 1 day at 37°C, and after being subjected to repeated loads, using the methods below.

### [Depth of cure]

Depth of cure was measured in compliance with ISO 4049:2009 after a 20-second light exposure with a dental LED photoirradiator (manufactured by Ultradent Products Inc. under the trade name VALO).

### [Cavity Sealing Properties after 1 Day at 37°C]

The surface of a human molar was cleaned with a brush under running water to obtain a sample with an enamel surface. A cylindrical cavity, measuring 4 mm in diameter and 5 mm in depth, was then formed with a dental diamond bur (manufactured by Mani Inc. under the trade name Mani Dia-Bur SF-21) by using an air turbine while applying water. Once formed, the cavity interior was rinsed with a stream of water, and was dried by blowing air. Thereafter, the self-adhesive dental composite resin was filled into the cavity so as to seal a depth of less than 1 mm in the cavity. After being left to stand for 10 seconds, the self-adhesive dental composite resin was irradiated with light using a dental LED photoirradiator (manufactured by Ultradent Products Inc. under the trade name VALO). After subsequent filling of the bulk-fill dental composite resin, the bulk-fill dental composite resin was cured by being exposed to light for 20 seconds using a dental LED photoirradiator (manufactured by Ultradent Products Inc. under the trade name VALO). The resultant sample was immersed in distilled water, and left to stand in this state for 24 hours inside a thermostatic chamber that had been set to 37°C. The sample was used as a test sample after being taken out of the chamber (n = 6).

Three of the six test samples were observed with an optical coherence tomography (OCT) device (IVS-2000 manufactured by Santec Corporation) to evaluate the adequacy of the cavities after 1 day at 37°C.

### <Evaluation Criteria>

Score 0: no detachment was observed in the cavities
Score 1: detachment was observed on side surfaces of cavities in any of the samples
Score 2: detachment was observed on the cavity floor in any of the samples
Score 3: detachment was observed on side surfaces and the floor of cavities in any of the samples

### [Cavity Sealing Properties after Repeated Loading]

For the evaluation of cavity sealing properties against repeated loads, the remaining three samples were subjected to compressive loads by repeatedly applying a load of 10 kgf to the center of the surface of the cured product of the dental composite resin filling the cavity, 100,000 times at 1 Hz frequency, using an indenter having a spherical tip. The samples were observed with an optical coherence tomography (OCT) device (IVS-2000 manufactured by Santec Corporation) to evaluate the adequacy of the cavities after the repeated loading, using the same criteria used for the evaluation of cavity sealing properties after 1 day at 37°C.

### [Comparative Examples 1 to 3]

The cavity sealing properties were evaluated after 1 day at 37°C and after repeated loading in the same manner as in Examples 1 to 9 using the same methods, except that a dental adhesive (manufactured by Kuraray Noritake Dental Inc. under the trade name Clearfil^{®} Tri-S Bond ND Quick) was used in a conventional fashion following the package insert, instead of using the self-adhesive dental composite resin (X). This dental adhesive is compositionally different from composite resins, and does not correspond to the self-adhesive dental composite resin (X).

### [Flexural Modulus]

The flexural modulus was evaluated by conducting a flexure test in compliance with ISO 4049:2009, specifically as follows. The prepared paste (a one-pack type self-adhesive dental composite resin composition) was filled into a SUS die (2 mm in length × 25 mm in width × 2 mm in thickness), and glass slides were pressed against the paste from the top and bottom (over a 2 mm × 25 mm surface). The paste was cured by applying light through the glass slides from both sides, 5 points on each side, 10 seconds each, using a dental LED photoirradiator (manufactured by Ultradent Products Inc. under the trade name VALO). The resultant sample was immersed in distilled water, and left to stand in this state for 24 hours inside a thermostatic chamber that had been set to 37°C. After being taken out of the chamber, the sample was used as a test sample for the measurement of flexural modulus. The flexural modulus was measured by conducting a three-point flexural test at a span length of 20 mm and a crosshead speed of 1 mm/min, using a universal testing machine (Autograph AG-I, 100kN, manufactured by Shimadzu Corporation) (n = 5), and a mean value was calculated.

Because the polymerization shrinkage stress alleviating effect tends to decrease, and there is a possibility of causing a decrease of cavity sealing properties when the flexural modulus of the cured product of the self-adhesive dental composite resin is too high, the preferred range of flexural moduli is 1.5 to 7 GPa, more preferably 2 to 6.5 GPa, even more preferably 3 to 6 GPa.

The flexural modulus was also evaluated for the bulk-fill composite resin using the same method.

**[Table 2]**

| Components (parts by mass) | | Composition 1 | Compositio n 2 | Compositio n 3 | Composition 4 | Composition 5 | Composition 6 |
|---|---|---|---|---|---|---|---|
| Monomer (a) having acidic group | MDP | 10 | 10 | 30 | - | - | 10 |
| | GPDM | - | - | - | 10 | 10 | |
| Monomer (b) having no acidic group | Bis-GMA | 30 | 15 | 30 | 30 | 15 | 30 |
| | D-2.6E | - | 15 | - | - | 15 | - |
| | 3G | 50 | 50 | 30 | 50 | 50 | 50 |
| | MAEA | 10 | 10 | 10 | 10 | 10 | 10 |
| Photopolymerization initiator (c-1) | Li-TPO | - | - | - | - | - | 0.3 |
| | CQ | 0.2 | 0.2 | 0.2 | 0.2 | 0.2 | 0.2 |
| Inorganic filler (d-1) | D-1 | 10 | 10 | 10 | 10 | 10 | 10 |
| | D-2 | 250 | - | 250 | 250 | - | 250 |
| | D-3 | - | 300 | - | - | 300 | - |
| Polymerization accelerator (e) | DABE | 0.4 | 0.4 | 0.4 | 0.4 | 0.4 | 0.4 |
| Other | BHT | 0.05 | 0.05 | 0.05 | 0.05 | 0.05 | 0.05 |
| Flexural modulus (GPa) | After 1 day at 37°C | 5.6 | 6.3 | 4.5 | 5.7 | 6.5 | 5.5 |

**[Table 3]**

| Components (parts by mass) | | Composition 7 | Composition 8 | Composition 9 |
|---|---|---|---|---|
| Monomer (b) having no acidic group | D-2.6E | 60 | 65 | - |
| | UDMA | 20 | - | - |
| | Bis-GMA | - | 10 | 75 |
| | 3G | 20 | 25 | 25 |
| Photopolymerization initiator (c-1) | TPO | 0.5 | - | 0.5 |
| | CQ | 0.2 | 0.3 | 0.2 |
| Polymerization accelerator (e) | DABE | 0.2 | 0.4 | 0.3 |
| Inorganic filler (d-1) | D-4 | - | 200 | - |
| | D-5 | 10 | - | 30 |
| | D-8 | 100 | - | - |
| | D-9 | - | 200 | 530 |
| Organic-inorganic composite filler (d-2) | D-10 | 30 | - | - |
| | D-11 | 170 | - | - |
| Flexural modulus (GPa) | After 1 day at 37°C | 7.3 | 10.2 | 16.5 |
| Depth of cure (mm) | | 5.1 | 5.4 | 5.7 |

**[Table 4]**

| | | Ex. 1 | Ex. 2 | Ex. 3 | Ex. 4 | Ex. 5 | Ex. 6 |
|---|---|---|---|---|---|---|---|
| Self-adhesive composite resin (X) | | Composition 1 | Composition 2 | Composition 3 | Composition 4 | Composition 5 | Composition 6 |
| Dental composite resin (Y) | | Composition 7 | Composition 7 | Composition 7 | Composition 7 | Composition 7 | Composition 7 |
| Flexural modulus ratio ((Y)/(X)) | | 1.3 | 1.2 | 1.6 | 1.3 | 1.1 | 1.3 |
| Cavity sealing properties | After 1 day at 37°C | 0 | 1 | 0 | 1 | 1 | 0 |
| | After repeated loading | 1 | 2 | 1 | 2 | 2 | 0 |

| | | Ex. 7 | Ex. 8 | Ex. 9 | Com. Ex. 1 | Com. Ex. 2 | Com. Ex. 3 |
|---|---|---|---|---|---|---|---|
| Self-adhesive composite resin (X) | | Composition 1 | Composition 3 | Composition 1 | - | - | - |
| Dental composite resin (Y) | | Composition 8 | Composition 9 | Composition 9 | Composition 7 | Composition 8 | Composition 9 |
| Flexural modulus ratio ((Y)/(X)) | | 1.8 | 3.7 | 2.9 | - | - | - |
| Cavity sealing properties | After 1 day at 37°C | 1 | 1 | 1 | 2 | 2 | 3 |
| | After repeated loading | 1 | 2 | 1 | 3 | 3 | 3 |

Compared to Examples 1 to 9, the cavity sealing properties after repeated loading on the cured product were inferior in Comparative Examples 1 to 3 in which the self-adhesive dental composite resin (X) was not used, as shown in Table 4.

### INDUSTRIAL APPLICABILITY

A dental filling kit of the present invention is useful because it shows good cavity sealing properties in a bulk-fill dental treatment of cavities deeper than 2 mm (particularly, 4 mm or more, or 5 mm or more), even when the cured product is subjected to repeated loads of impact from occlusion or other forces within the oral cavity.

## Claims

1. A dental filling kit comprising:
a self-adhesive dental composite resin (X) that comprises a monomer (a) having an acidic group, a monomer (b) having no acidic group, a polymerization initiator (c), and a filler (d); and
a dental composite resin (Y) that comprises a monomer (b) having no acidic group, a polymerization initiator (c), and a filler (d), but does not comprise a monomer (a) having an acidic group,
wherein the polymerization initiator (c) contained in the dental composite resin (Y) comprises a photopolymerization initiator (c-1), and
the dental composite resin (Y) is a bulk-fill composite resin, and has depth of cure of 4 mm or more.

2. The dental filling kit according to claim 1, wherein the content of the filler (d) is 50 to 90 parts by mass in total 100 parts by mass of the self-adhesive dental composite resin (X).

3. The dental filling kit according to claim 1 or 2, wherein a cured product of the self-adhesive dental composite resin (X) has a flexural modulus of 6.5 GPa or less.

4. The dental filling kit according to claim 3, wherein the self-adhesive dental composite resin (X) and the dental composite resin (Y) have a flexural modulus ratio ((Y)/(X)) of 0.9 to 5.0 as a ratio of a flexural modulus of a cured product of the self-adhesive dental composite resin (X) and a flexural modulus of a cured product of the dental composite resin (Y).

5. The dental filling kit according to any one of claims 1 to 4, wherein the monomer (a) having an acidic group contained in the self-adhesive dental composite resin (X) comprises a monomer having a phosphoric acid group.

6. The dental filling kit according to any one of claims 1 to 5, wherein the polymerization initiator (c) contained in the self-adhesive dental composite resin (X) comprises a photopolymerization initiator (c-1).

7. The dental filling kit according to claim 6, wherein the photopolymerization initiator (c-1) comprises a water-soluble photopolymerization initiator (c-1a) having a solubility in 25°C water of 10 g/L or more.

8. The dental filling kit according to any one of claims 1 to 7, wherein the monomer (b) having no acidic group contained in the dental composite resin (Y) comprises a (meth)acrylic acid ester (b-4) having an aromatic ring but having no hydroxyl group, and/or a (meth)acrylic acid ester (b-5) having an aromatic ring and a hydroxyl group.
